# EUROPEAN PATENT SPECIFICATION

(11) **EP 1 792 897 B1**
(45) Date of publication and mention of the grant of the patent: **16.09.2009**
(21) Application number: 07101154.8
(22) Date of filing: 23.06.1994
(51) Int. Cl.: C07D 221/12, C07D 417/06, C07D 413/12, C07D 219/14

(54) **Intercalators having affinity for DNA and methods of use**
Interkalatoren mit DNA-Affinität und Anwendungsverfahren
Intercalateurs ayant une affinité pour l'ADN et procédés d'utilisation

(30) Priority: 30.06.1993 US 86285
(43) Date of publication of application: 06.06.2007
(62) Divisional of application: 04001486.2
(73) Proprietor: ABBOTT LABORATORIES, Abbott Park IL 60064-3500 (US)
(72) Inventor: Bieniarz, Christopher, Highland Park, Illinois 60035 (US); Huff, Jeffrey, Bruce, Park Ridge, IL 60068 (US); Henrard, Denis, R., Ridgewood, NJ 07450 (US)
(74) Representative: Modiano, Micaela Nadia

(56) References cited:
- WO-A-93/08165
- US-A- 4 665 184
- US-A- 5 312 921
- GAUGAIN B ET AL: "DNA Bifunctional intercalators. Synthesis and conformational properties of an Ethidium homodimer and of an acridine ethidium heterodimer" BIOCHEMISTRY, AMERICAN CHEMICAL SOCIETY. EASTON, PA, US, vol. 17, no. 24, 28 November 1978 (1978-11-28), pages 5071-5078, XP002271620 ISSN: 0006-2960
- SCOTT C. BENSON ET AL: "Heterodimeric DNA-binding dyes designed for energy transfer : stability and applications of the DNA-complexes" NUCLEIC ACIDS RESEARCH, vol. 21, no. 24, 11 December 1993 (1993-12-11), pages 5720-5726, XP002431135 ARLINGTON, VIRGINIA US

## Description

### BACKGROUND

The present invention relates to intercalator compounds, and the use of such compounds, each of which is comprised of a substituted intercalator moiety, derivatized with a functionalized chain, which compounds have high affinity for binding to a DNA molecule. These intercalator compounds exhibit improved binding to a DNA molecule within known methodologies requiring intercalator insertion into the DNA molecule. Still, the invention relates to enhanced binding of DNA molecules by an intercalator-functioning segment utilized in labeling, capture, therapeutic insertion, assay and the like, with improved performance of the intercalator due to the increased utilization efficiency of the compounds.

The term "intercalator" was introduced into the chemistry field over 30 years ago to describe the insertion of planar aromatic or heteroaromatic compounds between adjacent base pairs of double stranded DNA (dsDNA). Many DNA intercalating compounds elicit biologically interesting properties. It is generally agreed that these properties are related to their reactivity with DNA. In the search for more active compounds, it is logical to design molecules with the highest possible affinity for DNA. In 1990, it was reported that complexes of ethidium homodimer with dsDNA performed at ratios of one dimer per four to five base pairs, and were stable to electrophoresis on agarose gels. This allowed fluorescence, detection and quantitation of DNA fragments with picogram sensitivity after separation and complete absence of background stain. Such a result has been sought through various manipulations of intercalator compounds, for example, by functional compounds made up of DNA intercalating dyes. As a result of these efforts, DNA intercalating agents utilizing ethidium bromide have been used in various DNA analytical procedures. ,

Various reported DNA intercalating agents utilizing ethidium bromide have been used in a multitude of DNA analytical procedures, for example:

Christen, et al., "An Ethidium Bromide-Agarose Plate Assay for the Nonradioactive Detection of CDNA Synthesis", Anal. Biochem., 178 (2), May 1, 1989, pp. 269-272, report ethidium bromide was used to determine the success of cDNA synthesis reactions. Since ethidium bromide in agarose can be used to quantitate RNA and DNA, conditions under which the greater fluorescence of double-stranded DNA is utilized were devised to assay double stranded DNA synthesis from mRNA. Ethidium bromide at 5 micrograms/ml in agarose allowed quantitative detection of cDNA in the range of 0.03 to 0.0015 microgram. Sodium dodecyl sulfate had an adverse effect on the measurement of cDNA. Subsequent cDNA analysis by alkaline gel electrophoresis and staining in 5 micrograms/ml ethidium bromide allowed accurate and rapid sizing of cDNA and required only 0.01-0.05 microgram cDNA.

Petersen, S.E., "Accuracy and Reliability of Flow Cytometry DNA Analysis Using a Simple, One-Step Ethidium Bromide Staining Protocol", Cytometry, 7 (4), July, 1986, pp. 301-306, reports that sources of variation and error were investigated for a simple flow cytometric analysis of DNA content of detergent-isolated nuclei stained with ethidium bromide.

In "Ethidium Bromide in the Detection of Antibodies to DNA and of Circulating DNA by Two-Stage Counterimmunoelectrophoresis", J.Immunol. Methods, 85 (1), Dec. 17, 1985, pp. 217-220, Riboldi, et al., report that in an attempt to overcome the limitations of counterimmunoelectrophoresis in the detection of precipitating anti-DNA antibodies or circulating DNA, ethidium bromide was used to increase the visibility of the precipitating lines and to confirm their specificity.

W.A. Denny reported in "DNA-Intercalating Ligands as Anti-Cancer Drugs: Prospects for Future Design", Anticancer Drug Des., 4 (4), Dec., 1989, pp. 241-263, that interest in DNA-intercalating ligands as anti-cancer drugs has developed greatly since the clinical success of doxsorubicin.

A number of agents have been described for labeling nucleic acids, whether probe or target, for facilitating detection of target nucleic acid. Suitable labels may provide signals detectable by fluorescence, radioactivity, colorimetry, X-ray diffraction or absorption, magnetism or enzymatic activity, and include, for example, fiuorophores, chromophores, radioactive isotopes, enzymes, and ligands having specific binding partners.

Fluorescent dyes are suitable for detecting nucleic acids. For example, ethidium bromide is an intercalating agent that displays increased fluorescence when bound to double stranded DNA rather than when in free solution. Ethidium bromide can be used to detect both single and double stranded nucleic acids, although the affinity of ethidium bromide for single stranded nucleic acid is relatively low. Ethidium bromide is routinely used to detect nucleic acids following gel electrophoresis. Following size fractionation on an approximate gel matrix, for example, agarose or acrylamide, the gel is soaked in a dilute solution of ethidium bromide.

The use of fluorescence labeled polynucleotide probes and polynucleotide hybridization assays have been reported. According to these methods, probes are prepared by attaching a particular absorber-emitter moieties to the three prime and five prime ends of the nucleic acid fragments. The fragments are capable of hybridizing to adjacent positions of a target DNA so that if both fragments are hybridized, the proximity of the absorber and emitter moieties results in the detectable emitter fluorescence. According to these methods, the fluorescent dye is introduced into the target DNA after all in vitro nucleic acid polymerizations have been completed. The inhibitory effects of intercalating agents on nucleic acid polymerases have been described in numerous locations.

DNA binding dyes are useful as antibiotics because of the inhibitory effects of nucleic acid replication processes that result from the agent binding to the template. The use of intercalating agents for blocking infectivity of influenza or herpes viruses have been reported. It has also been reported and described that a number of DNA binding agents, both intercalators and nonintercalators, inhibit nucleic acid replication. For example, ethidium bromide inhibits DNA replication.

Methods have been provided for detecting a target nucleic acid in a sample. These methods comprise the steps of (a) providing an amplified reaction mixture that comprises a sample, a DNA binding agent, where said agent is characterized by providing a detectable signal when bound to double stranded nucleic acid, which signal is distinguishable from the signal provided by said agent when it is unbound, and reagents for amplification; (b) determining the amount of signal produced by the mixture of step (a); (c) treating said mixture under conditions for amplifying the target nucleic acid; (d) determining the amount of said signal produced by the mixture of step (c); and (e) determining if amplification has occurred. These DNA binding intercalating agents, such as ethidium bromide or ethidium homodimer allow fluorometric study of the interaction of various molecules with DNA.

The intercalating agent useful for DNA binding or detecting amplified nucleic acids is an agent or moiety capable of insertion between stacked base pairs in the nucleic acid double helix. Intercalating agents such as ethidium homodimer and ethidium bromide fluoresce more intensely when intercalated into double stranded DNA than when bound to single stranded DNA, RNA, or in solution. Other uses of intercalators have been in the field of separation and isolation or purification of nucleic acids from complex biological or clinical specimens.

Various methods of separating deoxyribonucleic acids (DNA) from liquid biological samples are known in the art, but are very time consuming or otherwise plagued by complication. It is known that DNA adheres to nitrocellulose. The liquid sample containing DNA is applied to a nitrocellulose filter and the DNA adheres or binds to the filter.

Another method of separating DNA from samples is ultracentrifugation with sucrose or cesium chloride density gradients. The DNA is separated from other macromolecules such as proteins by this method according to the buoyant density or sedimentation coefficient. The biological sample is layered onto the density gradient in a centrifuge tube and is spun at very high speeds for long periods of time for DNA to travel through the density gradient. This method, although satisfactory, is very time consuming and labor intensive. The centrifugation time may be 20 hours or more per sample. Furthermore, if the sample is spun too long, the DNA will not only separate from the sample but also will pass entirely through the gradient to the very bottom of the centrifuge tube along with other constituents in the sample. Therefore, this method is also not suitable as a fast and easy method for separating DNA from complex samples.

Agarose polyacrylamide gel electrophoresis is also used to separate DNA from biological samples. In this method, the sample is applied to one end of a glass or plastic receptacle containing the gel and an electric current is applied across the length of the receptacle. The negatively charged nucleic acid molecules move toward the anode, the larger molecules moving more slowly. The rates of migration of the molecules depend on their molecular weights and on the concentration and degree of cross linking in the gel material. The DNA is then removed from the gel by cutting out that portion of the gel in which the DNA is located and finally extracting the DNA. Again, this method is time consuming and labor intensive, and the DNA must still be separated from the gel. When DNA is separated by the electrophoresis gel method or by centrifugation, it is necessary for the DNA to be stained in some manner to be visualized. Typically, ethidium bromide (EtBr) has been used as the staining agent. Ethidium bromide adheres to the DNA by intercalation between the base pairs of the double helix structure of the DNA.

More recently, an ethidium homodimer has been synthesized and introduced with bifunctional intercalators in order to allow fluorometric study including the interaction of such molecules with DNA. It has been determined that the ethidium homodimer ("EthD") binds to double stranded DNA ("dsDNA") about two (2) orders of magnitude more strongly than ethidium bromide. Complexes of EthD with dsDNA have performed at a ratio of one dimer per 4 to 5 base pairs and were found to be stable to electrophoresis on agarose base. On binding to dsDNA, the fluorescence quantum yield of the dimer increases 40 fold independent of nucleotide sequence.

Stable dsDNA-fluoropore complexes can be formed to obtain anywhere from several to several thousand fluoropores each, as desired. Under suitable controlled conditions these complexes do not transfer dye to other nucleic acids or proteins. An important property of these complexes is that their fluorescence emission intensity is a linear function of the number of intercalated dye molecules. As high sensitivity fluorescence detection apparatus becomes more generally available, the ability to use dyes to replace, for example, radioactivity for sensitivity detection of DNA, is becoming more and more valuable.

Dye dsDNA complexes represent a novel family of fluorescence labels with a wide range of spectroscopic properties whose composition, structure and size can be tailored to particular applications. DNA molecules can be readily derivatized to attach biotin, digoxigenin or any number of other substituents that can be recognized by avidin or antibodies. Such derivatized DNA molecules loaded with dye may allow detection at much higher sensitivity in numerous applications, for example, immunoassay, fluorescence, and in situ hybridization of chromosomes and the like that currently use other fluorescence labels.

Probes with a double stranded region, which provide intercalation sites and a single stranded region to allow recognition by hybridization of specific target sequences, offer another approach to the generation of versatile fluorescent labels. Development of conditions that allow clear discrimination between the binding of intercalators to single and double stranded nucleic acids is an essential prerequisite to the use of such probes.

Fluorescent probes are valuable reagents for the analysis and separation of molecules and cells. Some specific examples of their application are identification and separation from a subpopulation of cells in a mixture of cells by the techniques of fluorescence, flow cytometry, fluorescence-activated cell sorting, and fluorescence microscopy. Other applications include determination of a concentration of a substance or member of a specific binding pair that binds to a second species, or member of the specific binding pair, e.g., antigen-antibody reactions in an immunofluorescent assay. Still another application is the localization of substance in gels and other insoluble supports by the techniques of fluorescence staining.

Choice of fluorescers for these purposes is hampered by various constraints; one being the absorption and emission characteristics of the fluorescer since many ligands, receptors and other binding pair members, as well as other extraneous materials associated with the sample, for example, blood, urine and cerebrospinal fluid, will auto-fluoresce and interfere with an accurate determination or quantification of the fluorescent signal generated by the fluorescent label when the sample is exposed to the appropriate stimulus. Another consideration is the quantum efficiency of the fluorescer. Yet another concern is self-quenching; this can occur when the fluorescent molecules interact with each other when in close proximity. An additional concern is the non-specific binding of the fluorescer to other compounds or even with the test container.

It has been shown that dsDNA forms highly fluorescent complexes with the bis-intercalator EthD. Observations regarding the bis-intercalator EthD suggest that the intercalator can be exploited to generate a family of highly fluorescent stable dsDNA-dye complexes with distinctive properties. Such complexes could be exploited by multiplex detection of dsDNA fragments, as well as many analytical applications in which appropriately diversified dsDNA fragments labeled noncovalently with different dyes could be used as a unique family of fluorescent probes: 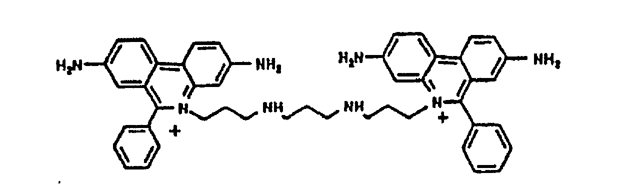 However, this compound may have a tendency to self-quench when bound to DNA.

In flow cytometry apparatuses, cells or other particles are caused to flow in a liquid flow stream so as to facilitate the investigation of certain characteristics thereof. In general, a flow cytometry apparatus is useful for identifying the presence of certain cells or particles of interest, enumerating those cells or particles and, in some instances, providing a sorting capability so as to be able to collect those cells or particles of interest. In a typical flow cytometry apparatus, a fluid sample containing cells is directed through the apparatus in a rapidly moving liquid stream so that each cell passes serially, and substantially one at a time, through a sensing region. Cell volume may be determined by changes in electrical impedance as each cell passes through the sensing region. Similarly, if an incident beam of light is directed at the sensing region, the passing cells scatter such light as they pass therethrough. This scattered light has served as a function of cell shape and size, index of refraction, opacity, granularity, roughness and the like. Further, fluorescence emitted by labeled cells, or autofluorescent cells, which have been excited as a result of passing through the excitation energy of the incident light beam is detectable for identification of cells having fluorescent properties. After cell analysis is performed by the flow cytometry apparatus, those cells that have been identified as having the desired properties may be sorted if the apparatus has been designed with such capability.

Instruments such as flow cytometry apparatuses are particularly useful for researchers and investigators studying various responses, reactions and functions of the immune system. Immunofluorescence studies, as well as fluorescence immunoassays, assist the investigator in identifying and targeting select cells of interest so that disease states, conditions and the like may be properly characterized. In addition to immune system investigations, fluorescence analysis is also quite beneficial in cell biology and morphology investigations, including the study of the substrate of cellular material.

In relying upon fluorescence to provide data and information about cells, the mechanics of performing tests for the fluorescence response is a major consideration in the design of the instrument as well as the results obtained. Specifically, the fluorescent markers, whether such markers be fluorescent stains or dyes, are typically excited by light energy. Usually there is an optimal wavelength which provides the greatest level of excitation for the fluorochromatic marker being used. Once excited, fluorescence emission occurs typically at wavelengths different from the wavelength of excitation. Fluorescence analysis instruments, whether fluorescence microscopes, image analyzers or flow cytometers, are generally designed to detect the fluorescence emission at the wavelength of emission maxima where the fluorescence signal is strongest.

Before the discovery and publication of the utilities of ethidium homodimer as an important intercalator, the usual intercalator of choice was ethidium bromide. Uses of the ethidium bromide intercalators include fluorometric methodologies, quantitative fluorescences of DNA intercalated ethidium bromide on agarose gels, ethidium bromide-agarose plate assay or detection of false DNA analysis and the like. Ethidium bromide and propidium bromide were further used in flow cytometry, as well as applications for direct electronic imaging, direct and rapid quantitation of fluorescence and electrophoretic gels in application as ethidium bromide-stain DNA. Ethidium bromide has also been used to increase the visibility of the precipitant lines and to confirm the specificity in two stage counter immunoelectrophoresis methodologies for detection of participating anti-DNA antibodies or circulating DNA. Utilization of ethidium bromide as an intercalator in numerous environments, as well as the more recent utilization of the ethidium homodimer intercalator are well documented in the literature and present the leading edge of intercalator methodology and efficiency.

In a somewhat different application of ethidium bromide as a staining agent, ethidium bromide has been linked to a solid support. U.S. Patent No. 4,119,521, issued to Chirikjian on Oct. 10, 1978, discloses a fluorescent DNA intercalating agent derivative of activated polysaccharides. The derivatives in the patent function as fluorescent stains to provide direct visualization of the DNA and their fractions, under the excitation of shortwave, ultraviolet radiation. The intercalating agents used in the patent are ethidium halides, with the preferred agent being ethidium bromide. This agent is coupled covalently to an activated polysaccharide such as agarose.

Utilization of ethidium bromide as an intercalator for use in numerous environments, as well as the more recent utilization of the ethidium homodimer intercalator are well documented in the literature and present the leading edge of intercalator methodology and efficiency. However, there remains an ever present need to improve utilization of the intercalators and viability of the use of intercalators with DNA, specifically addressing (1) high affinity for binding the intercalators to the DNA molecule; (2) reduction of self-quenching; and (3) providing superior transport kinetics. Intercalators possessing these qualities reduce the amount of intercalator required for performing one of the many functions involved in the aforementioned methodologies which can also enhance methodologies. In addition, improvement in accuracy and reliability of the various uses of interest is of continuing concern.

Gaugain, B et al, Biochemistry, vol. 17, no. 24, 28 November 1978, pages 5071-5078 disclose the synthesis and conformational properties of an ethidium homodimer and of an acridine ethidium heterodimer. WO 93/08165 discloses DNA-bis-intercalators. US Patent No. 4,665,184 discloses methidium or ethidium DNA intercalators linked to ethylene diamine tetraacetic acid. US Patent No. 5,312, 92I and Benson, S.C. et al, Nucleic Acids Research, 1993, vol. 21, No. 24, pages 5720-5726 disclose DNA intercalators having a fluorophore joined to a polycationic chain of at least two positive charges.

### SUMMARY

The present invention provides a compound having the formula 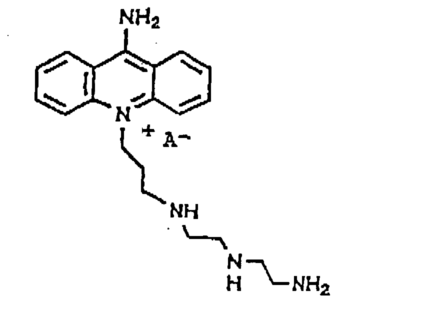 wherein A⁻ is an acceptable monovalent counter anion.

The present invention also provides a compound having the formula 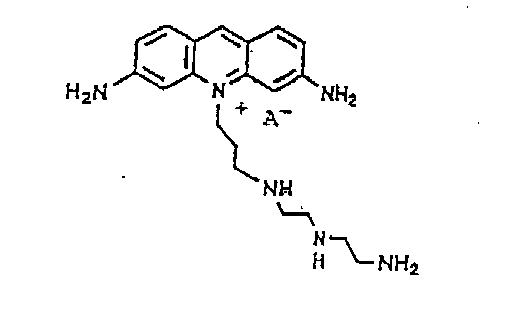 wherein A⁻ is an acceptable monovalent counter anion.

The above compounds provide a high affinity for binding to the DNA molecule and show reduced self-quenching while providing superior transport kinetics. The inventive intercalators have been found to provide enhanced fluorescence when bound to a DNA molecule within a fluorescent flow cytometry environment which is about eight to ten times brighter in fluorescence than ethidium homodimer utilized in the same flow cytometry environment. Because of the enhancement of fluorescence, the detection of DNA hybridization can be accomplished using much lower concentrations of intercalator compounds of the present invention than using conventional intercalating agents, such as ethidium homodimer or ethidium bromide. Using the same concentrations, intercalator compounds of the present invention can detect far less amounts of DNA hybridization than can conventional intercalating agents. Thus, the intercalator compounds of the present invention are far more sensitive than the known intercalating agents in detecting DNA hybridization.

Improvements in flow cytometry, fluorescence in-situ hybridization assays, gel electrophoresis, DNA detection, immunoassay for DNA, and other DNA studies are substantial. The use of intercalators with DNA and multiple methods have shown that the ethidium homodimer is about two orders of magnitude brighter than conventional staining methodology, i.e., ethidium bromide. However, the intercalator compounds in accordance with the present invention provide, for example, a dye which exhibits an eight to ten-fold increase in brightness over that of EthD in the same environment, or about a thousandfold improvement over more conventional staining methodologies. With pre-staining and post-electrophoresis detection, the sensitivity level of radioimmunoassay detection of DNA is now attainable with fluorophores. The compound compositions provided by this invention extends the limits of detection by up to tenfold over EthD, thereby providing a potential for new uses in applications of intercalators for the study of DNA analysis, as well as therapeutics and the like.

### BRIEF DESCRIPTION OF THE FIGURES

FIGURE 1 is a FACScan™ display for side scatter versus forward scatter;
FIGURE 2 is a histogram of fluorescence intensity (abscissa) versus frequency of events (ordinate);
FIGURE 3 is a scattergram representation for side scatter (SSC on abscissa) versus fluorescence intensity (ordinate);
FIGURE 4 is a FACScan™ display for side scatter versus forward scatter;
FIGURE 5 is a histogram of fluorescence intensity (abscissa) versus frequency of events (ordinate);
FIGURE 6 is a scattergram representation for side scatter (SSC on abscissa) versus fluorescence intensity (ordinate);
FIGURE 7 is a photographic representation of a UV light irradiated agarose electrophoresis gel performed on enzyme BAMH nicked PBR322 plasmid DNA;
FIGURE 8 is a hybridization saturation plot for equivalents;
FIGURE 9 shows hybridization titration curves for fluorescence intensity versus equivalents;
FIGURE 10A is a FACScan™ display (SSC vs FSC) of a typical distribution of lysed white cells;
FIGURE 10B is a FACScan™ display of the same blood sample as in FIGURE 10A with unstained chicken erythrocyte nucleii ("CEN") added;
FIGURE 10C is a FACScan™ display of the same sample lysed with white blood cell diluent ("WBC DIL");
FIGURE 10D is a FACScan™ display of the same sample as presented in FIGURE 10C, but with CEN;
FIGURE 10E is a FACScan™ display of the same sample lysed in the same diluent as FIGURE 10D, but with 0.5 µg/ml of nucleated red blood cell ("NRBC") dye;
FIGURE 10F is a FACScan™ display of the same sample lysed in the same diluent as FIGURE 10D, but with 0.25 µg/ml of NRBC dye;
FIGURE 11 is a graphical representation of the efficiency of 32P radiolabelled, restriction enzyme-nicked plasmid DNA capture onto phenathridinium activated polystyrene microparticles prepared as described in Example 6;
FIGURE 12 is a graphical representation of the efficiency of 32P radiolabelled plasmid DNA capture onto phenathridinium activated carboxymethyl sepharose beads synthesized as described in Example 6;
FIGURE 13 is a reaction scheme for the synthesis of compound 24 and its precursors;
FIGURE 14 is a schematic representation of intercalator derivatized solid phase microparticle;
FIGURE 15 is a reaction scheme for the synthesis of compound 85 and its precursors;
FIGURE 16 is a reaction scheme for the synthesis of compound 90 and its precursors;
FIGURE17 is a comparison of relative fluorescence intensities obtained from staining DNA by compound 24, ethidium bromide, propidium iodide, and ethidium homodimer.

### DETAILED DESCRIPTION

The present invention relates to the above-described intercalator compounds having high affinity for binding to a DNA molecule. Such intercalator compounds which exhibit improved binding to a DNA molecule within known methodologies requiring intercalator insertion into the DNA molecule. Enhanced binding of DNA molecules by an intercalator functioning segment is of utility in labeling, capture, therapeutic insertion, assay and the like, with improved performance of the intercalator due to the increased utilization efficiency of the compounds. Improved binding to the DNA molecule of the above intercalators is achieved, such intercalators exhibiting high affinity for binding, reduced self-quenching, and superior transport kinetics, especially when compared to ethidium homodimer or other bis-intercalators.

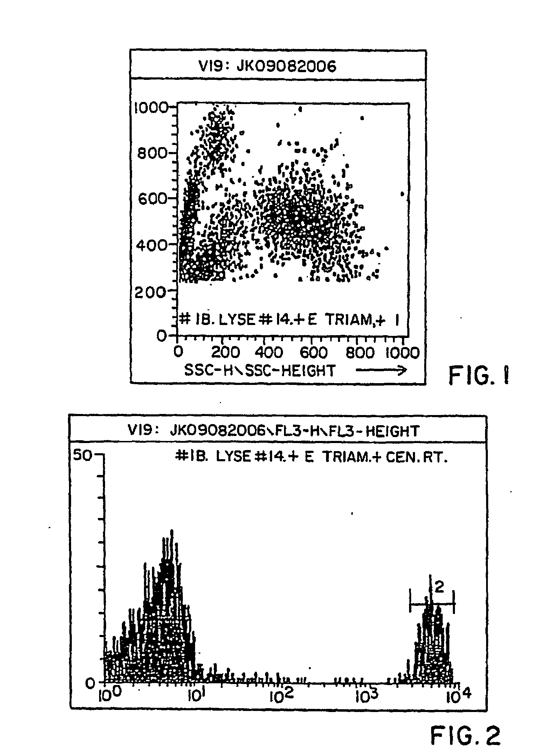

Synthesis of intercalator compounds and utilization of said compounds are shown in FIGURES 1-9, 10A-10F; 11-17. The information shown in these figures clearly demonstrates high affinity for binding, reduced self-quenching, and superior transport kinetics, especially when compared to ethidium homodimer or other bis-intercalators. The compounds of the invention are compounds 85 and 90.

FIGURE 1 is a FACScan™ display for side scatter versus forward scatter (SSC on abscissa axis and FSC on the ordinate axis) for a typical distribution of white cells lysed with WBC DIL diluent with NRBC dye phenathridinium triamine (PTA) 24 and CEN. The quadrant thresholds were set to preclude the lymphocytes gated on the SSC versus FSC dot plot.

FIGURE 2 is a histogram of fluorescence intensity (abscissa) versus frequency of events (ordinate) for the populations of stained and unstained cells in the presence of phenathridinium triamine (PTA) 24.

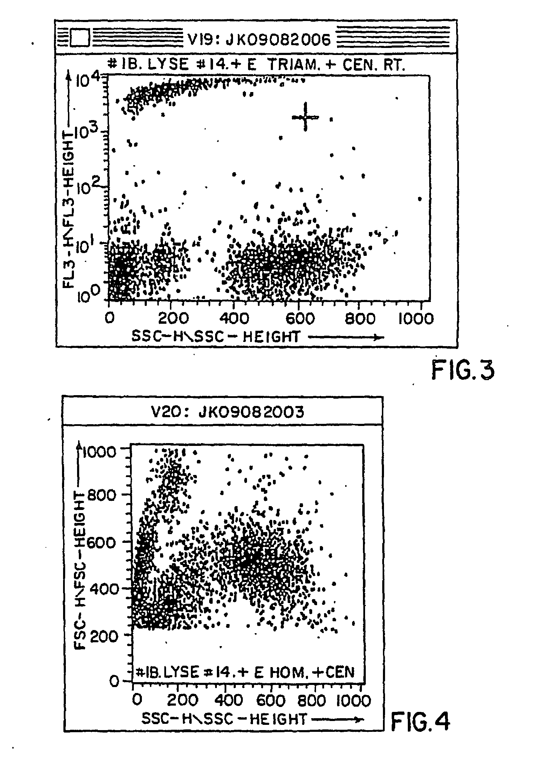

FIGURE 3 is a scattergram representation for side scatter (SSC on abscissa) versus fluorescence intensity (ordinate) showing the separation of cells stained with PTA 24 (upper left hand corner. NW quadrant) from unstained cells (remainder) by fluorescence intensity.

FIGURE 4 is a FACScan™ display for side scatter versus forward scatter (SSC on abscissa axis and FSC on the ordinate axis) for a typical distribution of white cells lysed with WBC DIL diluent with NRBC dye ethidium homodimer and CEN. The quadrant thresholds were set to preclude the lymphocytes gated on the SSC versus FSC dot plot.

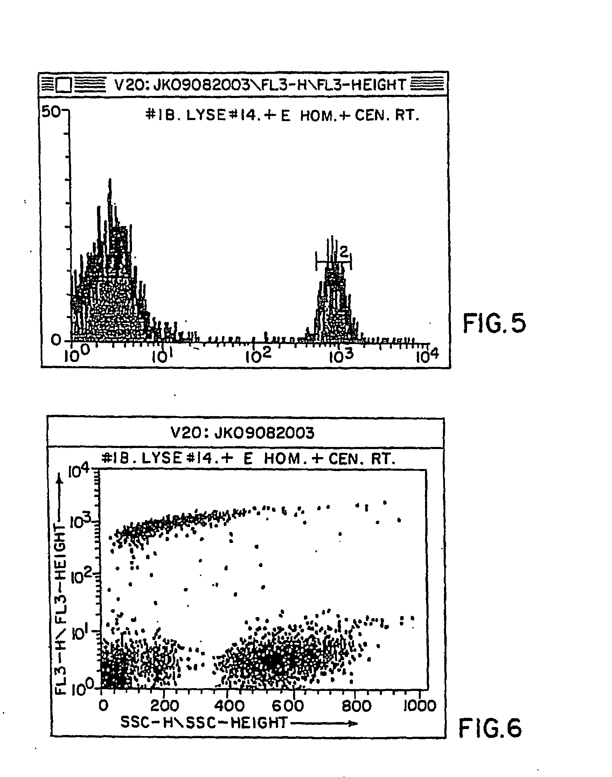

FIGURE 5 is a histogram of fluorescence intensity (abscissa) versus frequency of events (ordinate) for the populations of stained and unstained cells in the presence of ethidium homodimer.

FIGURE 6 is a scattergram representation for side scatter (SSC on abscissa) versus fluorescence intensity (ordinate) showing the separation of cells stained with ethidium homodimer (upper left hand corner, NW quadrant) from unstained cells (remainder) by fluorescence intensity.

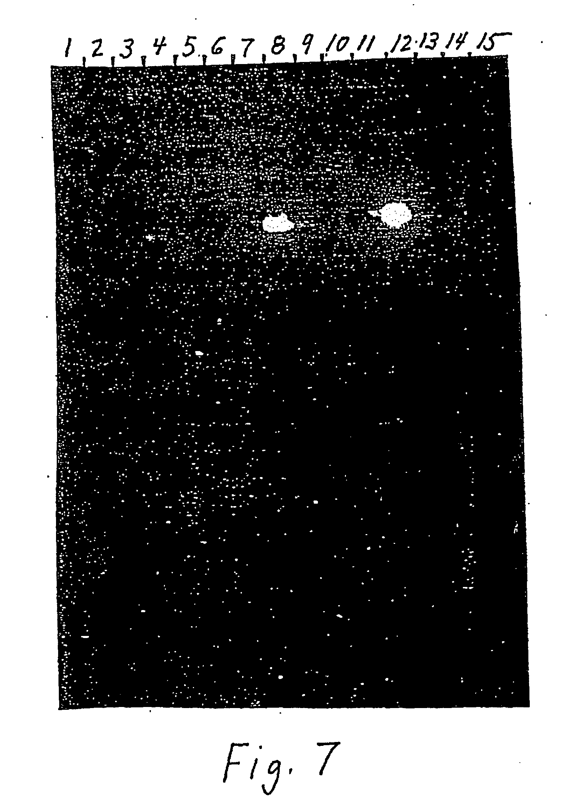

FIGURE 7 is a photographic representation of a UV light irradiated agarose electrophoresis gel performed on BAMH nicked PBR322 plasmid DNA. Loadings of 5 µl of stock solutions were made for lanes 3-14. The following stock solutions of DNA and intercalator were used to load lanes 1-14. Lane 1: marker; Lane 2: blank; Lane 3: 20 ng/ml plasmid stained with ethidium bromide; Lane 5: 160 pg/ml plasmid stained with ethidium bromide; Lane 6: 40 pg/ml plasmid stained with ethidium bromide; Lane 7: 20 ng/ml plasmid stained with ethidium homodimer; Lane 8: 800 pg/ml plasmid stained with ethidium homodimer; Lane 9: 160 pg/ml plasmid stained with ethidium homodimer; Lane 10: 40 pg/ml plasmid stained with ethidium homodimer; Lane 11: 20 ng/ml plasmid stained with PTA 24; Lane 12: 800 pg/ml plasmid stained with PTA 24; Lane 13: 160 pg/ml plasmid stained with PTA 24; Lane 14: 40 pg/ml plasmid stained with PTA 24. In all cases, the dye/base pair ratio was 1/20. In all cases, the dye was pre-incubated with the DNA and no post-gel-electrophoresis staining was done.

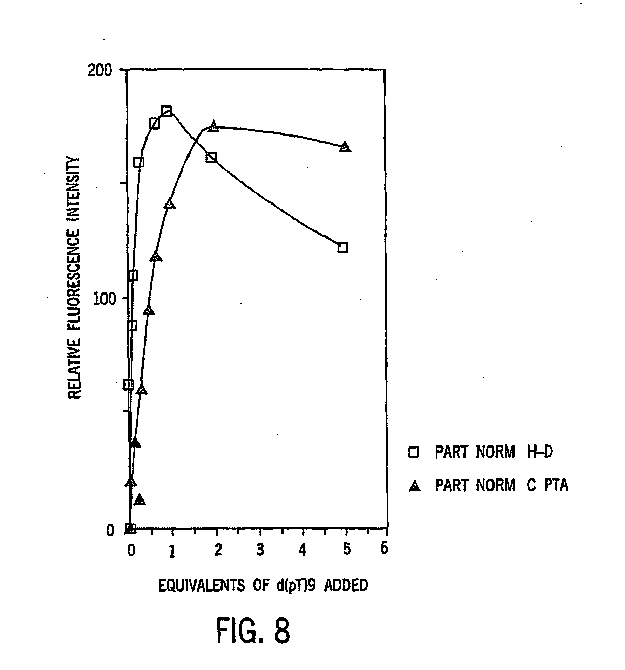

FIGURE 8 is a hybridization saturation plot for equivalents of d(pT)9 added to d(pA)9 at 6.6 micromolar DNA and 3.08 micromolar dye for ethidium homodimer and PTA 24 at a dye base pair ratio of 1:4. Graphical representation of equivalents of complementary oligonucleotide d(pT)9 on the abscissa added to d(pA)9 versus relative fluorescence intensity on the ordinate generated by using the protocol described in Example 2 to compare ethidium homodimer and PTA 24. The concentration of fluorophore in both cases was 3.08 micromolar using a two-fold statistical correction for the 2.0 molar equivalents of phenanthridinium moiety per each mole of ethidium homodimer. Both curves are normalized to background for relative fluorescence. Excitation was at 488 nm (534 nm is the excitation maximum) for this experiment and emission was at 625 nm.

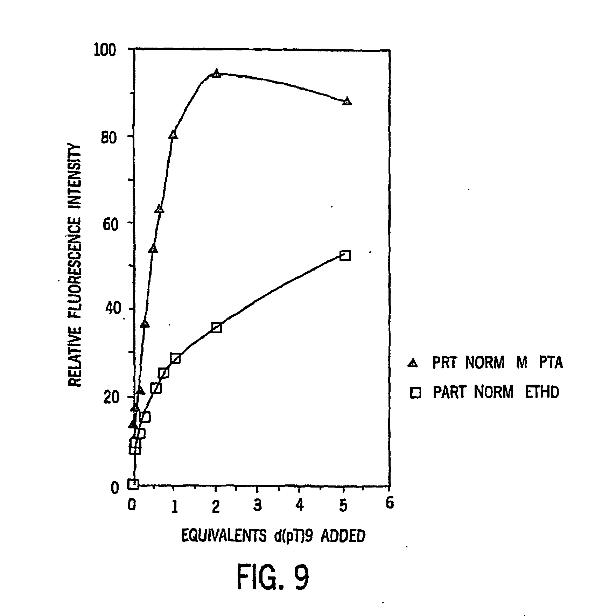

FIGURE 9 shows hybridization titration curves for fluorescence intensity versus equivalents of d(pT)9 added to d(pA)9 at 6.6 micromolar DNA and 3.08 micromolar ethidium bromide and PTA 24. Graphical representation of equivalents of complementary oligonucleotide d(pT)9 abscissa versus relative fluorescence intensity (ordinate) generated by using the protocol described in Example 2 to compare ethidium bromide and PTA 24. The concentration of fluorophore in both cases was 3.08 micromolar. Both curves are normalized to background. Excitation was at 488 nm (534 nm is maximum excitation) and emission was at 625 nm and intensities were measured using a Hitachi F-3010 fluorimeter.

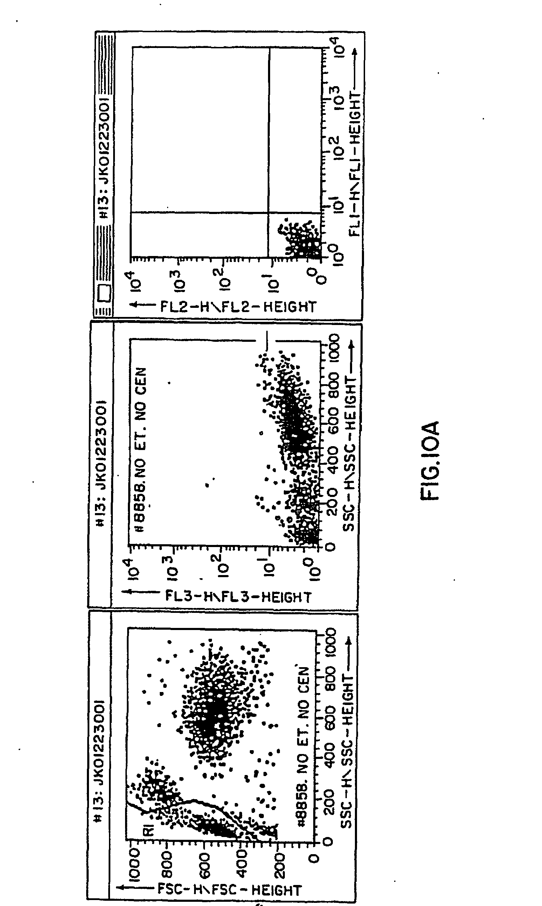

FIGURE 10A is a FACScan™ display (SSC vs FSC) of a typical distribution of white cells lysed with CD4000 WBC DIL without NRBC dye or CEN. The quadrant thresholds were set to preclude the lymphocytes gated on the SSC versus FSC dot plot.

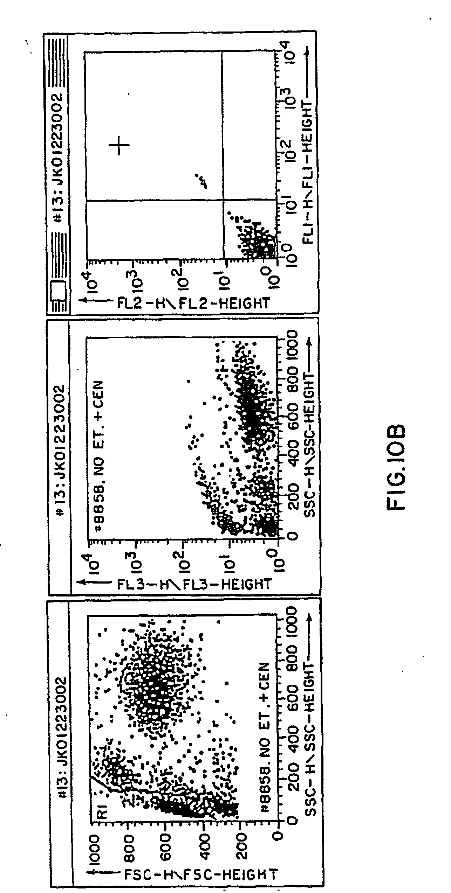

FIGURE 10B is a FACScan™ display of the same blood sample as in FIGURE 10A with unstained CEN added. As can be seen, the unstained CEN demonstrate some FRL3 auto-fluorescence. The region 1 was set to include all FL3+ events in this unstained sample, so that stained cells in the test samples can be counted in the region 2. The region 3 is set to include the CEN population only to measure the mean FL3 of the population.

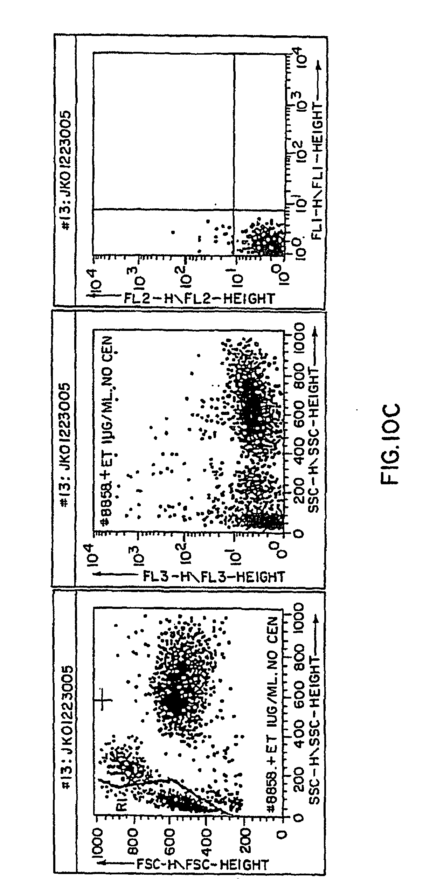

FIGURE 10C is a FACScan™ display of the same sample lysed with WBC DIL containing 1.0 ug/ml of NRBC dye. 1.3% of FL2+ events were detected in µL from the gated lymphocytes and 1.08% of FL3+ events are detected from the ungated total white cell population.

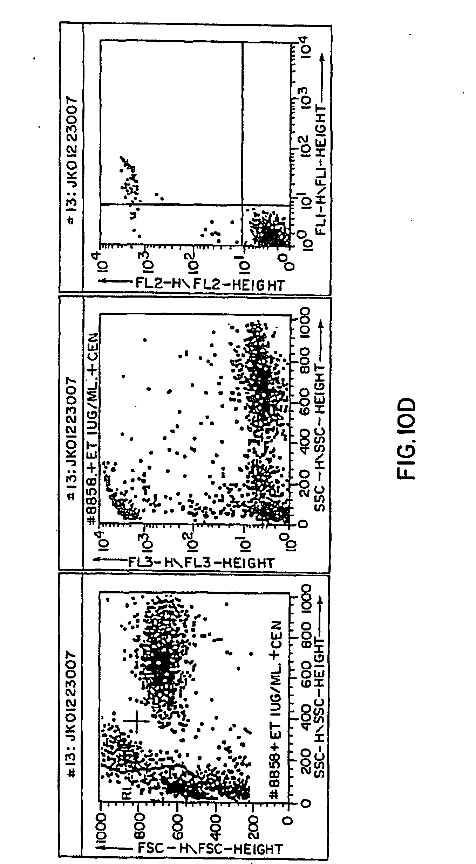

FIGURE 10D is a FACScan™ display of the same sample as presented in FIGURE 10C. but with CEN. The region 2 of the FL3 histogram of the ungated population shows the stained CEN population which has the mean FL3 of 3319.8.

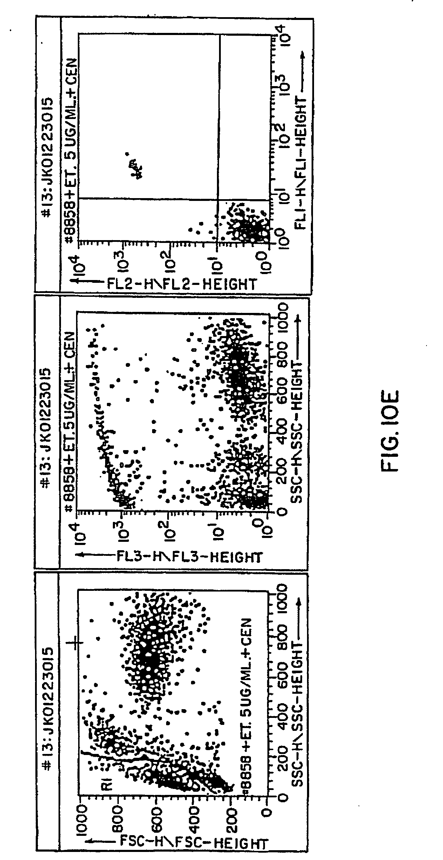

FIGURE 10E is a FACScan™ display of the same sample lysed in the same diluent as FIGURE 10D, but with 0.5 ug/ml of NRBC dye.

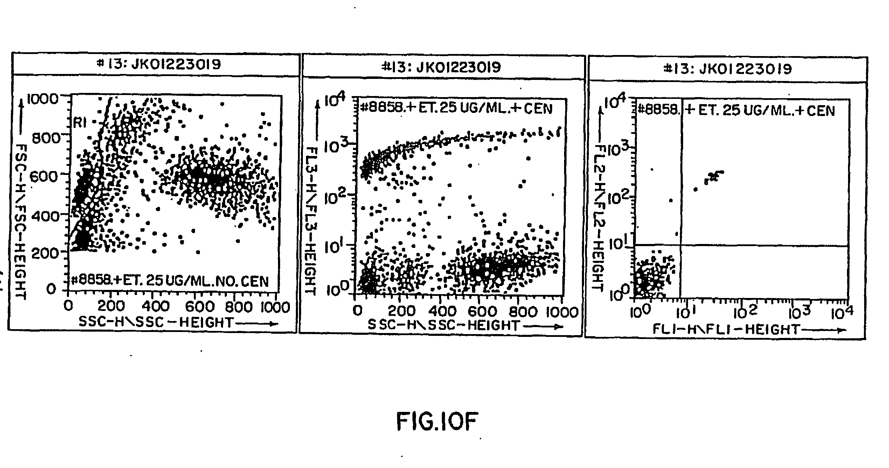

FIGURE 10F is a FACScan™ display of the same sample lysed in the same diluent as FIGURE 10D, but with 0.25 ug/ml of NRBC dye. As can be seen, the stained CEN is still well separated from the white cells.

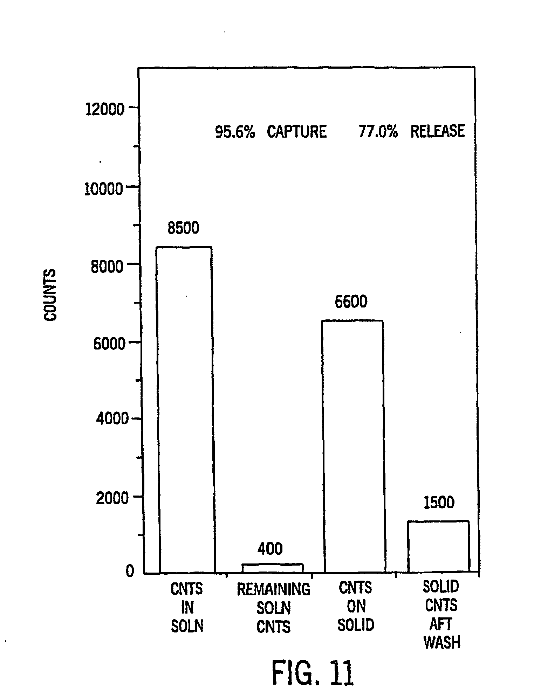

FIGURE 11 is a graphical representation of the efficiency of 32P radiolabelled plasmid DNA capture onto phenathridinium activated polystyrene microparticles synthesized as described in Example 6. A: initial radioactive counts in solution accounting for the total DNA concentration; B: radioactive counts remaining in solution after removal of DNA via centrifugation as described in Example 6; C: initial radioactive counts on the DNA bound to the microparticle by the phenthridine moiety before release is initiated by NaOH; and D: radioactive counts remaining on the solid after removal of DNA with NaOH.

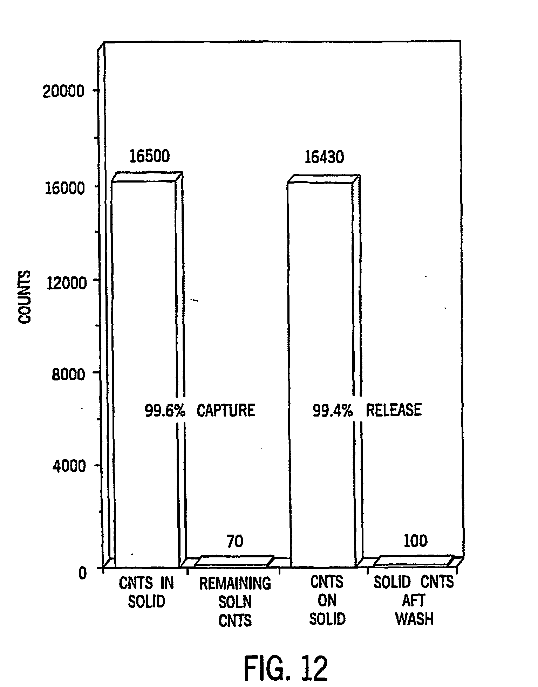

FIGURE 12 is a graphical representation of the efficiency of 32P radiolabelled plasmid DNA capture onto phenathridinium activated carboxymethyl sepharose beads synthesized as described in Example 6. A: initial radioactive counts in solution accounting for the total DNA concentration; B: radioactive counts remaining in solution after removal of DNA via centrifugation as described in Example 6; C: initial radioactive counts on the DNA bound to the microparticle by the phenthridine moiety before release is initiated by NaOH; and D: radioactive counts remaining on the solid after removal of DNA with NaOH.

The present intercalator compounds are substantially monointercalators, versus the polyfunctional (bis) intercalators.

In the following examples, only compounds 85 and 90 are intercalator compounds according to the invention. Examples relating to compound 24 are presented merely to illustrate the preparation and use of intercalator compounds.

### EXAMPLE 1

### Synthesis of Phenanthridinium Triamine (PTA) 24 And Precursor Intermediates 20-23

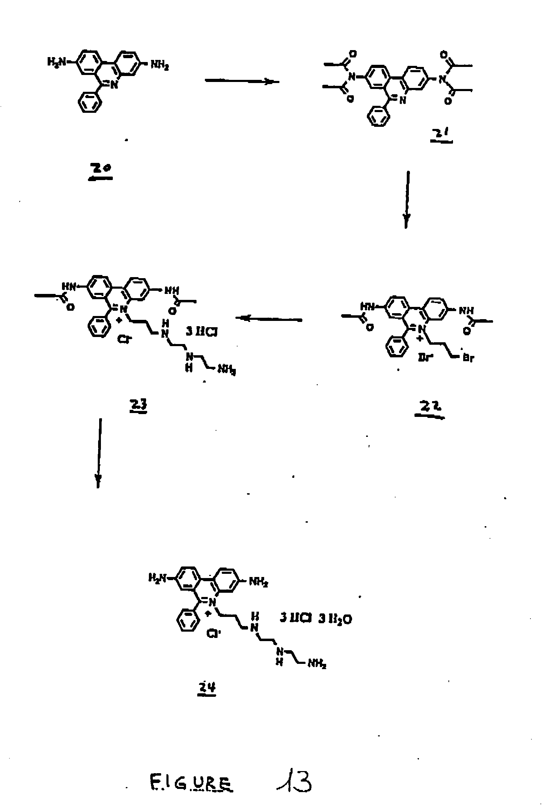

PTA 24 was synthesized through the sequence shown in the schematic as shown in FIGURE 13 - The experimental procedures used to obtain product 24 are as illustrated therein. Intermediate 21. Starting Intermediate 20, 3,8 Diamino 6-phenyl phenathridine (25.0 g, 0.0876 moles), was obtained from the Aldrich Chemical Company (Milwaukee, WI) and added to a single neck 3.0 liter round bottom flask under Argon and equipped with a magnetic stir bar and a reflux condenser. To this vessel, 1.0 liter of dry pyridine was added while stirring. Stirring of the resulting suspension was continued for 15 minutes until all the solid had dissolved. A catalytic amount of N,N-dimethylaminopyridine (1.07 g, 0.0086 moles) was added to this solution while stirring. Acetic anhydride (462 g, 4.9 moles) was then added and the resulting reaction mixture was refluxed for 8-12 hours. The reaction mixture was then allowed to cool and the solvent was removed in vacuo. For purification of the Product II, a gradient silica gel column was performed using 2.0 liters of 45/40/10/5 Ethyl acetate/hexane/ CH₂Cl₂CH₃OH followed by 1.0 liter of 40/40/10/10 EtOAc/hexane/ CH₂Cl₂/CH₃OH. Fractions of 10.0 ml were collected and appropriate fractions were recombined and the solvent was removed in vacuo. The sticky gum-like residue was then dissolved in hot EtOH (220 ml) and precipitated by cooling to 0° C. The mother liquor was decanted off and 200 ml of fresh EtOH was added. The solid was redissolved by heating and allowed to crystallize at -4° C for 48 hours. Crystals were collected from both the mother liquor and the second recrystallization and were washed with a small amount of cold EtOH and dried under high vacuum for several hours. Isolated yield of pure product after column chromatography and two recrystallizations was 32%. ¹H NMR CD₃OD (300 MHz) 9.1 (d, 1H 8.82 Hz), 9.0 (d, 1H, 8.75 Hz), 8.08 (s, 1H), 7.95 (s, 1H), 7.92 (d, 1H. 4.5 Hz), 7.8 (m, 3H), 7.65 (m, 3H), 2.45 (s, 6H), 2.35 (s, 6H); ¹³C NMR CD3OD (75.45 MHz) 174.5, 163.7, 145.3, 142.1, 140.6, 139.9, 134.4, 133.8, 130.8, 130.6, 129.8, 127.3, 125.9, 125.6, 124.8, 27.1, Exact Mass Calc. for C₂₇H₂₃N₃O₄, Calc. 453.1688 exact mass, Obs: 453.1683; CH analysis Calc for C₂₇H₂₃N₃O₄, C: 71.51 H: 5.11 N: 9.27 Found C: 71.77 H: 5.10 N:9.20.
Intermediate 22. Intermediate 22 was synthesized from the diamide 21 via a modification of a literature procedure (Gaugain et al., Biochemistry, Vol. 17, No. 24, 1978, pp. 5071-5078) for quarternization of the diamide of 3,8 Diamino-6-phenyl phenanthridine. Diamide 21 (10.5 g, 0.023 moles) was placed in a 2.0 liter round bottom flask under argon and equipped with a magnetic stir bar and reflex condenser. 1,3 Dibromopropane (1.0 liter, 9.86 moles) was added to this flask and the resultant mixture was brought to reflux for 7 hours. The solution was cooled overnight and the precipitant was filtered and washed with diethyl ether. Obtained 10.44 g (68.7%) of crude material 22. This material was recrystallized from CH₃OH to yield 5.3 g of diacetyl bromide 22. ¹H NMR CD₃OD (300 MHz) delta 10.75 (s, 1H), 10.45 (s, 1H), 9.3 (s, 1H), 9.09 (d, 9.2 Hz, 1H), 9.04 (d, 9.2 Hz, 1H), 8.45 (d, 9.1 Hz, 2.2 Hz, 1H). 8.12 (s, 1H). 8.07 (d 9.0 Hz, 1H), 7.95 (m, 3H), 7.85 (m, 3H), 5.0 (t, 9.0 Hz, 3H), 3.6 (t, 6.0 Hz, 2H), 2.65, 2.38 7.75 ¹³C NMR ₂₅DMSO [????] (75.45 MHz) delta 169.9, 169.3, 163.8, 142.1, 139.9, 134.2, 131.5, 130.5, 129.5, 128.4, 125.9, 125.4, 123.6, 122.3, 121.6. 119.0, 107.7, 55.7, 30.7, 24.5, 24.2; exact mass Calc. for C₂₆H₂₉N₃O₂Br₂ free salt (FAB+) 490.1131 Obs: 490.1139; CH analysis Calc. for C₂₆H₂₉N₃O₂Br₂ H: 4.41 C: 54.66 N:7.36 Found H: 4.25 C: 54.65 N: 7.30.
Intermediate 23. Intermediate 22 (5.3 g, 0.0093 moles) was added to a 250 ml round bottom flask equipped with a magnetic stir bar and reflux condenser. Methanol (150 ml) was then added to this flask while stirring under nitrogen and diethylene triamine (29.2 g, 0.283 moles) was added while stirring was continued. The resultant transparent solution was heated to reflux overnight under nitrogen. This solution was then allowed to cool to room temperature and poured into distilled H₂O. Then this mixture was concentrated in vacuo until only the H₂O remained. An additional 50-75 ml H₂O was added and the solution was allowed to cool to 0°C. The solid was then filtered and washed with ice cold water. This material was then redissolved in EtOH and precipitated with 10 N HCl. After filtration of the suspension, the resultant solid was recrystallized from hot ethanol upon cooling to 0°C for 15 minutes. A second crop was also collected from the second filtrate upon standing and by precipitation with EtOH from the first filtrate. These solids were then combined and the final product 23 (2.5 g) was obtained after high vacuum overnight. ¹H NMR CD₃OD (300 MHz) delta 9.2 (s, 1H). 9.05 (d, 1H), 8.95 (d, 1H). 8.4 (broad s, 2H), 8.3 (broad s, 1H), 7.9 (broad m, 3H), 7.7 (broad m, 2H), 5.1 (broad m, 1H), 3.9 (broad s, 3H), 3.45 (broad m, 2H), 3.85 (broad m, 2H), 2.5 (broad m, 3H), 2.3 (broad m, 3H); MS Calc. for free salt C₃₀H₃₇N₆O₂(FAB+) 513 Obs: 513.
Product 24. Synthesis and purification of the phenanthidinium triamine, PTA, 24 was accomplished by the following protocol. Triamine 23 (2.35 g, . 0.0036 moles) was dissolved in 75.0 ml methanol and 75 ml of 4N HCl was added. The mixture was refluxed for 2 hours and allowed to cool. Ethanol was added to this solution and resulting precipitate was filtered and washed with a minimal amount of cold ethanol. The filtrate was reconcentrated and fresh ethanol and concentrated aqueous HCl was added. This resulting precipitate was also filtered. Next, this filtrate was concentrated to near dryness and Et₂O was added and the solid filtered off. The last remaining unfilterable residue was then dissolved in concentrated HCl and precipitated with EtOH. This material was filtered and washed with ethanol, combined all solid materials from the above sequence and subjected this material to high vacuum overnight to obtain 2.03 g total of the high affinity fluorescent DNA stain PTA 24. ¹H NMR d₆-DMSO (300 MHz) delta 10.0 (broad s, 2H), 9.65 (broad s, 2H), 8.68 (d, 14.2 Hz, 2H), 8.35 (broad s, 2H), 7.75 (m, 4H), 7.65 (s, 1H) 7.55 (d, 9.2 Hz, 2H), 7.35 (d, 9.2 Hz, 2H), 6.28 (s, 1H). 4.5 (broad s, 2H), 4.0 (broad s. 8H), 3.4 (broad s, 2H), 3.0 (broad s, 2H), 2.3 (broad m, 2H); ¹³C NMR d₆-DMSO (75.45 MHz) δ 159.7, 151.1, 134.6, 131.7, 130.9, 129.4, 128.8, 128.4, 124.9, 122.9. 120.1. 117.4, 99.6, 51.4, 43.8, 42.5, 40.3, 34.9, 18.5; High resolution mass spec. C₂₆H₃₂N₆(FAB+) Calc. 429.2767, Obs: 429.2766; CH analysis Calc for C₂₆H₃₇N₆Cl₄.3H₂O was H 6.89 C 49.61 N 13.35 Found H: 6.16; C 49.74 N: 13.08.

### EXAMPLE 2

### Hybridization Assay

PTA 24 was used to quantitate hybridization when a target oligonucleotide was titrated with its complementary partner. A comparison of ethidium bromide staining versus PTA 24 for detecting this hybridization, as per the following protocol, can be found in FIGURE 9 and the comparison of ethidium homodimer versus PTA 24, as per the following protocol, can be found in FIGURE 8. Complementary strands of DNA (oligodeoxythymidylic acid, d(pT)₉ and oligodeoxyadenylic acid (d(pA)₉) were obtained from the Sigma Chemical Co. in SL Louis, MO. A stock solution of d(pA)9 was made at 5 units/ml of 0.05M TRIS, 0.2N NaCl, 1MM EDTA, pH 8.4. For polyA, ε=8.4 AU/mM cm or 8,400 M⁻¹cm⁻¹; therefore, with 9 base pairs for d(pA)9, the ε is 75,600 M⁻¹cm⁻¹. This stock was then diluted 10x to obtain stock at 6.61x10⁻⁶M, or 6.6 µM. The d(pT)ₚ stock was made at 25 units/5.0 ml and used for titration without further dilution in the same buffer. Since the ε for polyT is 8.15 AU/mM cm or 8,150 M-1cm-1 per base pair, or 73,350 M-1cm-1 per oligo, the concentration of the oligo stock was 68 µM in DNA molecules. A titration was performed using a Hitachi F-4010 Fluorescence Spectrophotometer equipped with 0.5 ml microcells to obtain fully corrected spectra and an excitation wavelength of 488-550 nm (optimal around 534) and an emission wavelength of 600-650 nm (optimal around 625). Equivalents of d(pT)₉ were added at the following increments: 0.02, 0.05, 0.080, 0.150, 0.300, 0.500, 0.700, 1.00, 2.00, 5.00 equivalents. Each sample in the titration curve was prepared individually by dividing the initial d(pA)9 stock into 10 x 1.0 ml aliquots. The addition of complement was then accomplished by micropipetting an appropriate amount (2, 5, 8, 15, 30, 50, 70, 100, 200, and 500 µl, respectively) of d(pT)9 stock to each of a series of the 10 aliquots. Each aliquot, obtaining progressively larger molar ratios of the two complementary strands, was incubated at ambient temperature for 15 minutes, the dye was added as 20.0 µl aliquots of a 154 µM solution of the dye in 0.05M TRIS, 0.2N NaCl, 1mM EDTA, pH 8.4 buffer. This corresponds to a dye/DNA b.p. ratio of 1/20 at saturation with complementary oligo. Overall concentrations of dye and oligo vary in the saturation plot because of the use of varied increments additions from the same stock solution. After an additional 15 minute incubation time, the relative fluorescence intensity was then read at 625 nm and recorded to generate a standard curve which is directly proportional to the quantity of dsDNA hybridization, or target sequence, under these conditions. The background fluorescence, or initial residual fluorescence, is then subtracted out as a constant for all curves for comparison of the various titration curves on the same graph.

### EXAMPLE 3

### Gel Electrophoresis Application

An agarose gel was run to compare the staining intensity of ethidium bromide (Aldrich Chemical Co., Milwaukee, WI), ethidium homodimer -I (Molecular Probes, Cat. # E 1169, Eugene, Oregon), and PTA 24 stain. Plasmid, pBR322, at 2.1 mg in 7 ml stock was incubated at 37°C for 1 hour with 1 ml of BAMH restriction enzyme with 2 ml 10X React2 Buffer and diluted to 20 ml total with 10 ml H₂O. This mixture was then used to prepare 3 stocks of nicked pBR322 plasmid at 0.63 mg per 6 ml for each vial. Each of these stocks were diluted further with H₂O and 20% glycerol to final DNA stocks of 20 ng/ml. 800 pg/ml. 160 pg/ml, and 40 pg/ml with a 1:4 ratio of dye to DNA base pairs in each for a total of 12 stocks. A 5 ml aliquot of each stock was loaded into 12 separate lanes in agarose gel and electrophoresis was run for 30 minutes in 4 mM TRIS, pH8.2, with 0.01mM EDTA buffer. The gel was then removed and photographed under exposure to U.V. light in a conventional gel box.

### EXAMPLE 4

### Protocol For Synthesis Of Intercalator Activated Carboxymethyl Styrene Microparticle Capture Reagent

The synthesis of intercalator derivatized solid phase microparticle (MP) capture reagent was accomplished by the scheme depicted in the following schematic and effected by the following procedure:

A 45 aliquot of 0.275 ± µm microparticles (Seradyne, Indianapolis, IN) were placed in a 4 ml vial and the surfactant was exchanged out using Bio-Rex 501-D ion exchange mixed bed resin (Bio-Rad, Richmond, CA). After gentle shaking for 2 hours, the resin was filtered out from the mixture by using a coarse fritted glass funnel equipped with a reduced pressure collection chamber. The sample was diluted to a concentration of MP at 10% solids by weight. The total amount of equivalents of reactive carboxylic acid were calculated from the titration specifications of the vendor.

A stock solution of sulfo N-hydryoxysurcinimide (Pierce, Rockford, IL) was made at I1 mg/ml (20 mM) in H₂O and a stock solution of EDAC (Sigma chemical Co., St Louis, MO) at 10 mg/ml (5 mM) was made in H₂O. Five equivalents of EDAC (290 µl stock) was added to the carboxymicroparticle reaction mixture, followed by 5.0 equivalents of sulfo N-hydryoxysuccinimide (330 µl stock). This mixture was allowed to incubate at room temperature for 2 hours and then a 2.0 molar equivalent of PTA 24 (4 mg) was added at a concentration of 8 mg/400 µl, or 2.0 mg/100 µl in pH 8.0 0,1 N NaCl 0.1N Pi phosphate buffer. N-hydryoxysucciaimide (Pierce) can be substituted for sulfo N-hydryoxysuceinimide if it is first dissolved in a stock of DMF (Dimethyl formamide) and aliquoted as described above. After allowing 24 hours for complete reaction, the free dye was then removed by centrifugation, removal of mother liquor, and resuspension for several attempts until the solution went clear and no more dye was extracted from the samples. The purified capture reagent was then diluted to a stock of 2-4% solids in H₂O.

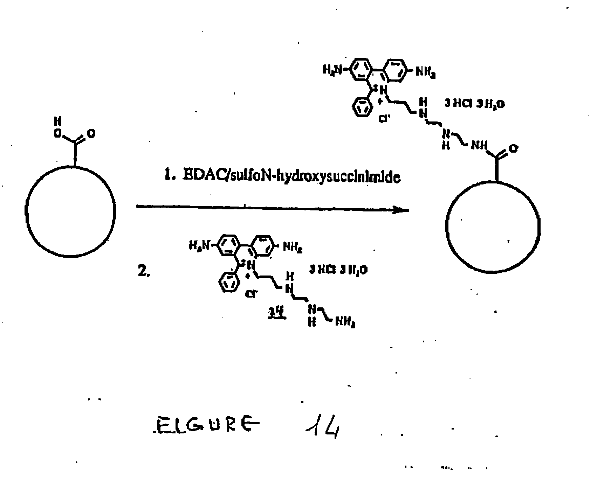

A general schematic representation of this Example is given in FIGURE 14,

### EXAMPLE 5

### Solid Phase DNA Capture

CM (carboxy modified) Sepharose was obtained from Stigma Chemical Co. (St Louis, MO) in an ethanol/H₂O mixture. The solution was estimated at 50% solids based on total volume occupied by the solid and liquid portions on extended standing. This suspension was then mixed uniformly and diluted to 10% solids. 200 µl of this stock was removed and calculated at 0.12 meq/gram to be 0.012 meq of acid total. Stock of EDAC and N-hydryoxysuccinimide were prepared and 5.0 equivalents of each activating reagent were added to this suspension. For this preparation. 13.2 mg (in 1.32 ml) HOSuc and 11.25 mg EDAC (in 1.02 ml) were used and 8.0 mg total of the PTA 24 intercalator. After incubation for 2-24 hours, the suspensions were then cleaned by repeated washing and gentle centrifugation steps until no more color was removed from the solid upon dilution. A stock was prepared at 10% solids in H₂O. Note that controls were run with PTA 24 modified and non-modified solid phases and minimal non-specific capture of DNA occurred with the underivatized materials.

### EXAMPLE 6

### Protocol For DNA Capture By Intercalator Modified Solid Phase

1. Place 50 µl of activated microparticles in a 1.5 ml eppendorf.
2. Add 150 µl of PBS and 1-20 u of a 5.0 kb linearized plasmid end-labeled with ³²P. Alternatively, added 1-50 µl of biological sample or another purified DNA.
3. Mix by rotation for one hour at ambient temperature.
4. Pellet the microparticles by centrifugation at 5,000 rpm for 5 minutes.
5. Wash one or two times with 200 µl of PBS.
6. Release the DNA by adding 50 µ of 0.5 M NaOH and mix for 15 minutes at ambient temperature.
7. Centrifuged and collected the supernatant, which contains released DNA.

The efficiency of DNA capture was measured using ³²P radiolabelled plasmid DNA in the above-described protocol. The results are found in FIGURE 11 using the intercalator modified polystyrene microparticles prepared as per Example 4 and FIGURE 12 and using the intercalator modified CM Sepharose beads prepared per Example 5. The data indicates that the DNA binding to the intercalator modified solid phase was specific and induced by the covalent attachment of intercalator 24 to the solid phase.

### EXAMPLE 7

### Relative Staining Intensities Of Ethidium Homodimer And Phenanthridinium Triamine 24 In A Flow Cytometric Study Of Chicken Erythrocyte Nuclei (CEN)

Protocol: 50 µl of whole blood sample from two in-house donors and 3µl of CEN suspension was added to 1.0 ml of pre-warmed at 40°C WBC DIL without and with the NRBC dye at 1 µg/ml concentration, mixed, introduced to the FACScan™ and 20" readings were acquired. Chicken erythrocyte nuclei (CEN) were used to measure the brightness of the FL3 staining (mean FL3 of CEN). The whole blood samples used were about 4-5 hours old. The data for this experiment is shown in FIGURES 1-6.

### EXAMPLE 8

### Comparative Performance Of Reduced Phenathidinium Triamine 24 Dye Concentrations Relative To Ethidium Homodimer

The effect of a reduction in PTA 24 dye concentration (FIGURES 10A-F) relative to ethidium homodimer was demonstrated as follows:

Method: The experiment was designed to show the correlation between the dye concentration and the percent of FL2+ events in the UL quadrant on the FL1 versus FL2 dot plots. The WBC DIL used contained 0.5% weight/volume of ammonium chloride, 0.075% of volume of formaldehyde, 0.01% weight/volume of saponin, 0.01% weight/volume of potassium bicarbonate, and 20 mM acetate buffer with a pH of about 6.0 and an osmolality of about 270 mOsm per liter. 50 µl of whole blood sample from each of two in-house donors was added to 1.0 ml of pre-warmed at 40°C WBC DIL without and with the NRBC dye of varying concentration (0.25, 0.50, 0.75, and 1.0 ug/ml), mixed, introduced to the FACScan™ and 20" readings were acquired. Chicken erythrocyte nuclei (CEN) suspension was used to measure the brightness of the FL3 staining (mean FL3 of CEN). The whole blood samples used for this experiment were about 6 hours old.

It was observed that the CEN DNA is essentially indistinguishable from the background without the PTA 24 (FIGURE 10B) and that the dye concentration can be reduced to 75% of that of ethidium homodimer (FIGURE 5) and still maintain acceptable separation from the background (FIGURE 10F). Such a reduction can lead to significantly reduced non-specific binding and substantial savings in dye usage.

### EXAMPLE 9

### Viability Dyes On The Coulter Elite™ Flow_Cytometer

Cell Isolation Protocol: Each tube of ficol isolated cells were treated as follows: PBS with 0.1% NaAzide and 1.0% albumin (Sigma catalogue #1000-3) Ficol specific gravity 1.119 (Sigma Histopague catalogue #1119-1),

10 ml of whole blood (EDTA anticoagulant) was diluted with 10 ml of PBSW. Into 4, 15 ml conical bottom tubes, 5 ml of the diluted blood was layered over 5 ml of ficol. The tubes were spun for 30 minutes at 400 x G. The interface layer which contains the lymphocytes, monocytes, granulocytes and platelets was aspirated and washed once in 5 ml PBS, by centrifuging tubes at 300 x G for 6 minutes. The cell pellet was resuspended in PBS, cells counted, and adjusted to 8.5 x 106 cells per ml.

### Cell Staining: Protocol:

### Dye solutions:

PTA 24 - Stock solution 10 ug/ml made by dissolving PTA 24 in PBS with 0.1% NaAzide.

Propidium iodide (P.I.) - Stock solution 0.5 mg/ml made by dissolving P.I. in PBS with 0.1 % NaAzide.

### P-I. Staining:

In 12 x 75 mm tube, 117.6 µl of cells was mixed gently with 14.7 µl of P.I. stock dye solution. After 20 seconds, the tube was placed on a Coulter Elite™ flow cytometer and data collected.

Procedure from "Discrimination of Viable and Non-Viable Cells Using Prodidium Iodide in Two Color Immunofluorescence", Cytometry by Sasaki et al., Vol. 8, 1987, pp. 413-420.

### PTA 24 Staining:

In 12 x 75 mm tube, 23.5 µl of cells was gently mixed with 76 µl of PTA 24 stock dye solution. After 20 seconds, the tube was placed on a Coulter Elite™ flow cytometer and data collected.

### Trypnan Blue Staining:

In 12 x 75 mm tube, 5 µl of working solution Trypan Blue and 5 µl of cells were gently mixed and cells counted in a mehacytometer using standard white light illumination. A minimum of 500 cells were counted within 3 minutes of staining.

Procedure from Selected Methods in Immunology by Mishell and Shiigi, 1980, pp. 16-17.

### Flow cytometer protocol: Cells analyzed on the Elite™ flow cytometer (Coulter Electronics, Inc.).

Samples were excited with an argon laser at 488 nm and 15 mW of power. Data was gated on the basis of size and granularity to exclude red blood cells, platelets and debris. The linear dye fluorescence of the gated distribution was analyzed using unstained cells as a control. The percent positive events (dead cells) and the mean fluorescence of the dead cell distribution were recorded.

**TABLE I**

| Viability Dyes on the Coulter Elite™ Cytometer | | |
|---|---|---|
| Time Point | Sample | % Positive (Dead Cells) |
| 5 hr | P.I. | 2.5 |
| | PTA | 2.2 |
| | Trypan Blue | 1.4 |
| 27 hr | P.I. | 6.3 |
| | PTA | 7.7 |
| | Trypan Blue | 4.8 |
| 103 hr | P.I. | 26.8 |
| | PTA | 19.1 |
| | Trypan Blue | 10.2 |

### EXAMPLE 10

### Synthesis Of Compound 85 And Its Related Compounds

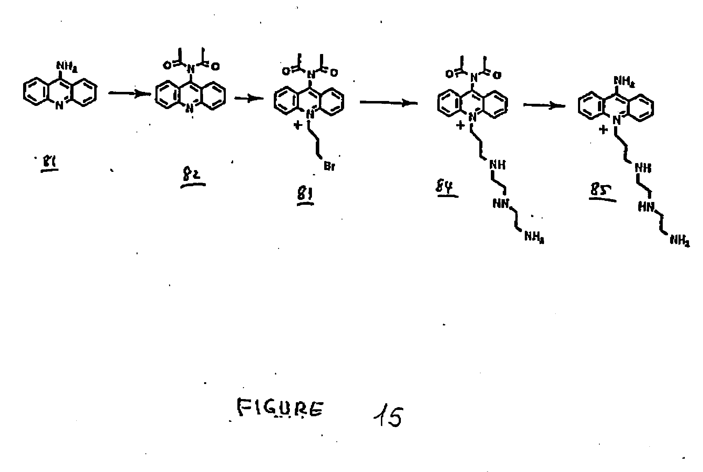

The reaction scheme for the general synthesis of compound 85 and its precursors is given in FIGURE 15.

Starting material 81 (0-0376 males) is obtained from the Aldrich Chemical Company (Milwaukee, WI) and is added to a single neck 3.0 liter round bottom flask under Argon and. equipped with a magnetic stir bar and a reflux condenser. To this vessel 1.0 liter of dry pyridine is added while stirring. Stirring of the resulting suspension is continued for 15 minutes until all the solid is dissolved. A catalytic amount of N.N-dimethylaminopyridine (1.07 g, 0.00876 moles) is added to this solution while stirring. Acetic anhydride (462 g, 4.9 moles) is then added and the resulting reaction mixture is refluxed for 8-12 hours. The reaction mixture is then allowed to cool and the solvent is removed in vacuo. For purification of the product 42, a gradient silica gel column is performed using an appropriate solvent system determined using methods well known to those skilled in the art such as Thin Layer Chromatography. Fractions of 10.0 ml are collected and appropriate factions are recombined and the solvent is removed in vacuo. The residue is then dissolved in hot EtOH (220 ml) and precipitated by cooling to 0°C. The mother liquor is decanted off and 200 ml of fresh EtOH is added. The solid is redissolved by heating and is allowed to crystallize and -4°C for 48 hours. Crystals are collected from both the mother liquor and the second recrystalization and are washed with a small amount of cold EtOH and dried under high vacuum for several hours. The resultant compound 82 can be characterized by high resolution mass spectrometry as well known to those skilled in the art.

Compound 83 can be synthesized from the imide 42 via a modification of a literature procedure of Gaugain, et al., Biochemistry, Vol. 17, No. 24, 1978, pp. 5071-5078 for quarternization of the diamide of 3,8 diamino-6-phenyt phenanthridine. Imide 82 (0.023 moles) is placed in a 2.0 liter round bottom flask under Argon and is equipped with a magnetic stir bar and reflux condenser. 1,3-Dibromopropane (1.0 liter, 9.86 moles) is added to this flask and the resultant mixture is brought to reflux for about 7 hours. The solution is cooled overnight and the precipitant is filtered and washed with Et₂O. This material is recrystallized from CH₃OH to yield diacetyl bromide 83 which can be characterized by mass spectrometry and other methods known to those skilled in the art.

Compound 83 (0.0081 moles) is added to a 250 ml round bottom flask equipped with a magnetic stir bar and reflux condenser. Methanol (150 ml) is then added to this flask while stirring under nitrogen and the diethylene triamine (0.283 moles), which is available from the Aldrich Chemical Company is added while stirring is continued. The resultant solution is heated to reflux overnight under nitrogen. This solution is then allowed to cool to room temperature and is poured onto distilled H₂O. Then, this mixture is concentrated in vacuo until only the H₂O remains. An additional 50-75 ml H₂O is added and the reaction mixture is cooled to 0°C. The solid is filtered and washed with ice cold water. This material is then redissolved in EtOH and precipitated with 10 N HCl After filtration of this suspension, the resultant solid is recrystallized from hot ethanol upon cooling to 0°C for about 15 minutes. A second crop is also collected from the second filtrate upon standing and by precipitation with EtOH from the first filtrate. These solids are then combined and the product 84 can be obtained and can be subjected to high vacuum overnight Characterization can be effected by calculating the molecular mass of the free base amine from the exact isotopic mass formulas weD known to those skilled in the art and comparing the resultant mass with that obtained by a high resolution mass spectrometry molecular weight determination such as are well known to those skilled in the art.

Synthesis and purification of the final product 85 can be accomplished by the following protocol. The imide (0.0036 moles) 84 is dissolved in 75.0 ml methanol and 75 ml of 4N HCl is added. The mixture is refluxed for about 2 hours and allowed to cool. Ethanol is added to this solution and resulting precipitate is filtered and washed with a minimal amount of cold ethanol. The filtrate is reconcentrated and fresh ethanol and concentrated aqueous HCl is added. This resulting precipitate is also filtered. Next, this filtrate is concentrated to near dryness and Et₁O is added and the solid filtered off. The last remaining unfilterable residue is then dissolved in concentrated HCl and precipitated with EtOH. This material is filtered and washed with Ethanol. All solid materials are combined from the above sequence and subjected to high vacuum overnight to obtain 85. Characterization can be effected by calculating the molecular mass of the free base amine from the exact isotopic mass formulas well known to those skilled in the art and comparing the resultant mass with that obtained by a high resolution mass spectrometry molecular weight determination such as arc well known to those skilled in the art.

### EXAMPLE 11

### Synthesis Of Compound 90 And Its Related Compounds

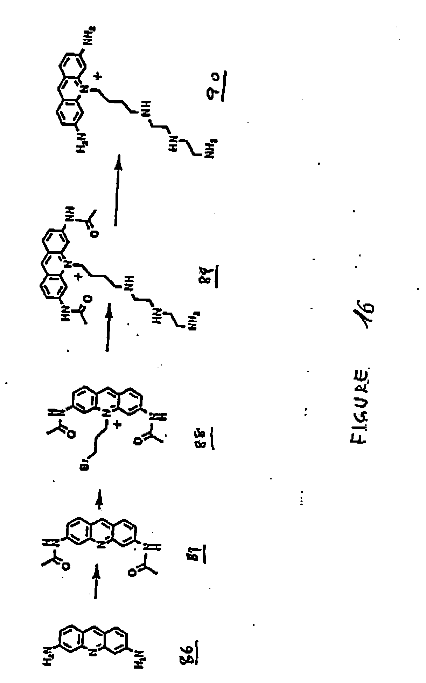

The reaction scheme for the general synthesis of compound 90 and its precursors is given in FIGURE 16.

Starting material 86 (0.0876 moles) is obtained from the Aldrich Chemical Company (Milwaukee, WI) and is added to a single neck 3.0 liter round bottom flask under Argon and equipped with a magnetic stir bar and a reflux condenser. Acetic anhydride (462 g, 4.9 moles) is then added and the resulting reaction mixture is refluxed for 8-12 hours. The reaction mixture is then allowed to cool and the solvent is removed in vacuo. For purification of the product 87, a gradient silica gel column is performed using an appropriate solvent system determined using methods well known to those skilled in the art. Fractions of 10.0 ml are collected and appropriate fractions are recombined and the solvent is removed in vacuo. The residue is then dissolved in hot EtOH (220 ml) and precipitated by cooling to 0°C. The mother liquor is decanted off and 200 ml of fresh EtOH is added. The solid is redissolved by heating and is allowed to crystallize and -4°C for 48 hours. Crystals are collected from both the mother liquor and the second recrystalization and are washed with a small amount of cold EtOH and dried under high vacuum for several hours. The resultant compound 87 can be characterized by high resolution mass spectrometry as well known to those skilled in the art.

Compound 88 can be synthesized from the diamide 87 via a modification of a literature procedure of Gaugain, et al., Biochemistry, Vol. 17, No. 24, 1978, pp. 5071-5078 for quarternization of the diamide of 3,8-diamino-6-phenyl phenanthridine. Diamide 87 (0.023 moles) is placed in a 2.0 liter round bottom flask under Argon and is equipped with a magnetic stir bar and reflux condenser. 1,3-Dibromopropane (1.0 liter, 9.86 moles) is added to this flask and the resultant mixture is brought to reflux for about 7 hours. The solution is cooled overnight and the precipitant is filtered and washed with Et₂O. This material can be recrystallized from CH₃OH to yield diacetyl bromide 88 which can be characterized by mass spectrometry and other methods known to those skilled in the art.

Compound 88 (0.0081 moles) is added to a 250 ml round bottom flask equipped with a magnetic stir bar and reflux condenser. Methanol (150 ml) is then added to this flask while stirring under nitrogen and the diethylene triamine (0.283 moles), which is available from the Aldrich Chemical Company, is added while stirring is continued. The resultant solution is heated to reflux overnight under nitrogen. This solution is then allowed to cool to room temperature and is poured onto distilled H₂O. Then, this mixture is concentrated in vacuo until only the H₂O remains. An additional 50-75 ml H₂O is added and the reaction mixture is cooled to 0°C. The solid is filtered and washed with ice cold water. This material is then redissolved in EtOH and precipitated with 10 N HCl. After filtration of this suspension, the resultant solid is recrystallized from hot ethanol upon cooling to 0°C for 15 minutes. A second crop is also collected from the second filtrate upon standing and by precipitation with EtOH from the first filtrate. These solids are then combined and the product 89 can be obtained and is subjected to high vacuum overnight. Characterization can be effected by calculating the molecular mass of the free base amine from the exact isotopic mass formulas well known to those skilled in the art and comparing the resultant mass with that obtained by a high resolution mass spectrometry molecular weight determination such as are well known to those skilled in the art.

Synthesis and purification of the final product 90 can be accomplished by the following protocol The diamide (0.0036 moles) 89 is dissolved in 75.0 ml methanol and 75 ml of 4N HCl is added. The mixture is refluxed for 2 hours and allowed to cool. Ethanol is added to this solution and resulting precipitate is filtered and washed with a minimal amount of cold ethanol. The filtrate is reconcentrated and fresh ethanol and concentrated aqueous HCl is added. This resulting precipitate is also filtered. Next, this filtrate is concentrated to near dryness and Et₂O is added and the solid filtered off. The last remaining unfilterable residue is then dissolved in concentrated HCl and precipitated with EtOH. This material is filtered and washed with Ethanol. All solid materials are combined from the above sequence and subjected to high vacuum overnight to obtain compound 90. Characterization can be effected by calculating the molecular mass of the free base amine from the exact isotopic mass formulas well known to these skilled in the art and comparing the resultant mass with that obtained by a high resolution mass spectrometry molecular weight determination such as are well known to those skilled in the art.

### EXAMPLE 12

### Hybridization Assay Using Compound 24

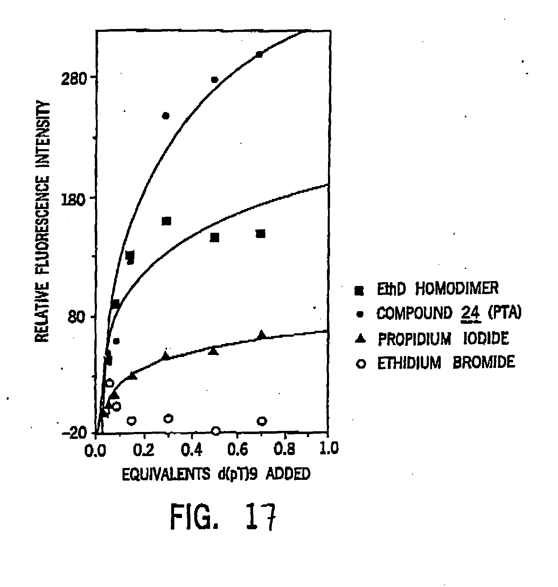

In one experiment PTA 24 was used to quantitate hybridization when a target oligonucleotide was titrated with its complementary partner. By also conducting three simultaneous but independent experiments, a comparison of ethidium bromide staining, propidium iodide staining and ethidium homodimer staining verus PTA 24 was made as follows. Results can be found in FIGURE 17.

Complementary strands of DNA oligodeoxythymidylic acid, d(pT),, and oligodeoxyadenylic acid, (d(pA),), were obtained from the Sigma Chemical Co. in St. Louis, MO. A stock solution of d(pA), was made at 5.0 units/0.5 nd of 0.004 M TRIS, 0.001 M EDTA, pH 8.2 buffer. For poly A, ε=8.4 AU/mM cm or 8400 M⁻¹cm⁻¹; Therefore, with 9 base pairs for d(pA),, the ε is 75,600 M⁻¹cm^{-1.} This stock was then diluted 100X to obtain stock at 6.61x10⁻¹M, or 0.66 µM. The d(pT)9 stock was made at 25 units/5.0 ml and used for titration after dilution 10X with the same buffer. Since the ε for polyT is 8.15 AU/mM cm or 8,150 M⁻¹cm⁻¹ per base pair, or 73,350 M⁻¹cm⁻¹ per oliogo, the concentration of the oligo stock was 6.8 µM in DNA molecules. A titration was performed using a Hitachi F-4010 Fluorescence Spectrophotometer using polystyrene disposable 4.0 ml cuvettes to obtain a fully corrected spectra and an excitation wavelength of 488-550 nm (using 488 mn for this experiment) and an emission wavelength of 600-650 nm (using 625 nm for this experiment). Equivalents of d(pT), were added at the following increments: 0.020, 0.080, 0.150, 0.300, 0.500, 0.700, 1.000, 2.000, 5.000 equivalents. Each sample in the titration curve was prepared individually by dividing the initial d(TA), stock in 10 x 1.0 ml increments. The addition of complement was then accomplished by micropipetting an appropriate amount (2, 5, 8. 15, 30. 50. 70, 100, 200, and 500 µl, respectively) of d(pT)9 Stock to each of a series of the 10 aliquots. Each aliquout, containing progressively larger molar ratios of the two complementary strands, was incubated a ambient temperature for 1 hr 45 minutes, the corresponding dye was added as 100.0 µl aliquots of a 15 µM solution of the corresponding dye in 0.004M TRIS, 0.001M EDTA, pH 8.2 buffer. This corresponds to a dye/DNA b.p. ratio of 1/4 at saturation with complementary oligo. Overall concentrations of dye and oligo vary in the saturation plot because of the use of varied increments additions from the same stock solution. After an additional 45 minutes incubation time after addition of the corresponding appropriate dye, the relative fluorescence intensity was read at 625 nm and recorded to generate a standard curve which is directy proportional to the quantity of dsDNA hybridization, or target sequence, under the same conditions. The background, or initial residual fluorescence, is then substrated out as a constant for all curves for comparison of the various titration curves on the same graphs.

It is apparent to those skilled in the art that the high binding affinity of the intercalator compound 24 is especially useful relative to conventional intercalators such as ethidium bromide (FIGURE 17). In FIGURE 17 the sensitivity or response to ds-DNA concentration in the presence of intercalator compound 24 is shown compared to ethidium bromide, propidium iodide and ethidium homodimer. When concentrations of oligonucleotide are less than 10⁻⁶M, the advantages of such high affinity intercalators is especially apparent.

In comparison of PTA 24 to homobifunctional intercalators such as ethidium homodimer (FIGURE 17), again a clear advantage is sensitivity is observed. In this case, the increased sensitivity may be related to reduced self-quenching in the PTA 24 as well as its high affinity for DNA.

In FIGURE 8 and FIGURE 9, the concentration of ds-DNA was higher and the differential between the ethidium bromide and PTA 24 (FIGURE 9) and ethidium homodimer and PTA 24 (FIGURE 8) was not significant because of the higher concentration of DNA used.

In summary, the present invention offers clear advantages in allowing the detection of ds-DNA hybridization at much lower concentrations than conventional staining methods currently available or known in the art.

### EXAMPLE 13

### Hybridization Assay /Using Intercalator Compound 24

DNA Intercalator 24 can be used to quantitate hybridization when a target oligonucleotide is titrated with its complementary partner. Complementary strands of DNA oligodeoxythymidylic acid, d(pT), and oligodeoxyadenylic acid, (d(pA),), can be obtained from the Sigma Chemical Co. in St. Louis, MO. A stock solution of d(pA)9 is made at 5 units/ml of 0.05M TRIPS, 0.2N NaCl, 1mM EDTA, pH 8.4 or other suitable buffer. For polyA, ε=8.4 AU/mM cm or 8,400 M⁻¹cm⁻¹; therefore, with 9 base pairs for d(pA)9, the ε is 75,600 M⁻¹cm⁻¹. This stock is then diluted to obtain stock from 0.066 - 66 µM. The d(pT), stock is made at 25 units/5.0 ml and used for titration without further dilution in the same buffer. Since the for polyT is 8.15 AU/mM cm or 8,150 M-1cm⁻¹ per base pair, or 73,350 M-1cm⁻¹ per oligo, the concentration of the oligo stock is 0.0068 - 660 µM in DNA molecules. A titration can be performed using a Fluorescence Spectrophotometer using an excitation wavelength of 488-550 nm (optimal around 534) and an emission wavelength of 600-650 nm (optimal around 625). Equivalents of d(pT), is added at the following increments: 0.02, 0.05, 0.080, 0.150, 0.300, 0.500, 0.700, 1.00, 2.00, 5.00 equivalents. Each sample in the titration curve is prepared individually by dividing the initial d(pA)9 stock into 10 x 1.0 ml aliquots. The addition of complement is then accomplished by micropipetting an appropriate amount (2, 5, 8, 15, 30, 50, 70, 100, 200, and 500 µl, respectively) of d(pT)9 stock to each of a series of the 10 aliquots when the d(pT)9 stock is 10X the concentration of the d(pA)9 stock. Each aliquot, obtaining progressively larger molar ratios of the two complementary strands, is incubated at ambient temperature for 0.25 - 2.5 hours, the dye is added as 1-500 µl aliquots of a 1- 1000 µM solution of the dye in 0.05M TRIS, 0.2N NaCl, 1mM EDTA, pH 8.4 buffer or other suitable buffer. This corresponds to a dye/DNA b.p. ratio of 1/1 - 1/1000 at saturation with complementary oligo. Overall concentrations of dye and oligo vary in the saturation plot because of the use of varied increments additions from the same stock solution. After an additional 15 minute incubation time, the relative fluorescence intensity is then read at between 580 - 680 nm and recorded to generate a standard curve which is directly proportional to the quantity of dsDNA hybridization, or target sequence, under these conditions.

### EXAMPLE 14

### Determination of Hybridization of Complementary Binding Pairs Other Than d(pT)9 and d(dA)9 Using Intercalator Compound 24.

By using the above described procedure from EXAMPLE 13, intercalator compound 24 can be used to determine the quantity of hybridization of any other complementary DNA strands by substituting d(pT)9 with the appropriate complementary DNA and substituting d(pA)9 with the appropriate target DNA at appropriate concentrations that are determined by one skilled in the art and depending on the degree of complementary regions that are expected as is determined by one skilled in the art. In each case, the excitation wavelength of 450-550 nm (optimal at 534 nm) can be used and the relative fluorescence intensity is then read at between 580 - 680 nm and recorded to generate a standard curve which is directly proportional to the quantity of dsDNA hybridization, or target sequence, under these conditions.

### EXAMPLE 15

### Protocol for Synthesis of Intercalator Activated Carboxymethyl Styrene Microparticle Capture Reagent Using Intercalator Compound 85 or 90

The synthesis of intercalator derivatized solid phase microparticle (MP) capture reagent can be accomplished by the following procedure:

A 45 aliquot of 0.275 ± µm microparticles (Seradyne, Indianapolis, IN) is placed in a 4 ml vial and the surfactant is exchanged out using Bio-Rex 501-D ion exchange mixed bed resin (Bio-Rad, Richmond, CA). After gentle shaking for 2 hours, the resin is filtered out from the mixture by using a coarse fritted glass funnel equipped with a reduced pressure collection chamber. The sample is diluted to a concentration of mp at 10% solids by weight. The total amount of equivalents of reactive carboxylic acid is calculated from the titration specifications of the vendor. A stock solution of sulfo N-hydryoxysuccinimide (Pierce, Rockford, IL) is made at 11 mg/ml (20 mM) in H₂O and a stock solution of EDAC (Sigma Chemical Co., St. Louis, MO) at 10 mg/ml (5 mM) is made in H₂O. Five equivalents of EDAC (290 µl stock) is added to the carboxymicroparticle reaction mixture, followed by 5.0 equivalents of sulfo N-hydryoxysuccinimide (330 µl stock). This mixture is allowed to incubate at room temperature for 2 hours and then a 2.0 molar equivalent of intercalator compound

85 or 90 (4 mg) is added at a concentration of 8 mg/400 µl, or 2.0 mg/100 µl in pH 8.0 0.1 N NaCl 0.1N Pi phosphate buffer. N-hydryoxysuccinimide (Pierce) can be substituted for sulfo N-hydryoxysuccinimide if it is first dissolved in a stock of DMF (Dimethyl formamide) and aliquoted as described above. After allowing 24 hours for complete reaction, the free dye is then removed by centrifugation, removal of mother liquor, and resuspension for several attempts until the solution went clear and no more dye is extracted from the samples. The purified capture reagent is then diluted to a stock of 2-4% solids in H₂O.

### EXAMPLE 16

### Solid Phase DNA Capture using Solid Phase Derived From Compound 85 or 90

The capture of DNA onto solid phase can be performed using derivatization methods as described in EXAMPLE 5 except substituting the appropriate solid phase synthesized from the respective intercalator 85 or 90 as described in EXAMPLE 15.

Other appropriate solid phases such as carboxylated polystyrene microparticles or carboxylated magnetic microparticles can are also used instead of CM Sepharose.

### EXAMPLE 17

### Protocol for DNA Capture by Intercalator Modified Solid Phase Modified By Compound 85 or 90

DNA can be released from solid phase as described in EXAMPLE 6 except that corresponding solid phase derived from intercalator compound 85 or 90 is used rather than the PTA 24 derivatized intercalator.

### EXAMPLE 18

### Conjugates of Intercalator Derivatized Antibodies and Intercalator Derivatized Alkaline Phosphatase Using Double Stranded Nucleic Acids as Conjugation Templates

Complementary strands of oligonucleotides, sense and antisense 14 to 20-mers, can be synthesized on a fully automated DNA synthesizer and purified as described in Bioconjugate Chemistry, 4, pp, 94-102, (1993). The synthesis of the conjugate between the enzyme calf intestinal alkaline phosphatase and IgG can be accomplished as follows.
a) Derivatization of the Intercalator PTA 24 with 30 Atom Heterobifunctional Linker Arm, 4-[(*N*-maleimidomethyl)tricaproamid o]cyclohexane-1-carboxylate (SMTCC).
   PTA 24, 0.100g (2.3x10⁻⁴ mole), is dissolved in 5 ml of 50% aqueous DMF (dimethylformamide). 30 atom heterobifunctional linker, 4-[(*N*-maleimidomethyl)tricaproamido]cyclohexane-1-carboxylate (SMTCC), 0.157 g, (2.3x10⁻⁴ mole) synthesized as described in Bieniarz et al., US Patent Nos. 4,994,385, 5,002,883, 5,053,520, and 5,063,109 is dissolved in 3 ml of DMF and is added in one portion to the solution and stirred over 24 h at ambient temperature. The resulting maleimide derivatized PTA 24 contains is purified on silica gel column using 10% methanolic acetone as eluent.
b) Derivatization of the Calf Intestinal Alkaline Phosphatase with Iminothiolane.
   Calf intestinal alkaline phosphatase, 6 mg (4x10⁻⁹ mole) in 1 ml of PBS buffer is thiolated by treatment with a 450-fold molar excess of iminothiolane (164 ml of a 15 mg/ml solution in PBS buffer) for 30 min at ambient temperature. Excess reagent is removed by gel filtration with a Sephadex G-25 column (1x45 cm) equilibrated with PBS buffer. The fractions containing the derivatized enzyme are pooled and the concentration of the enzyme in the pool is calculated by absorbance at 280 nm.
c) Conjugation of the SMTCC Derivatized PTA 24 to the Thiolated Calf Intestinal Alkaline Phosphatase from b).
   The thiolated calf intestinal alkaline phosphatase from part b) and maleimide derivatized PTA 24 from part a) are combined at molar ratio 1:1 and incubated 18 h at 5°C. Unreacted thiol groups are capped by addition of 5 mM *N*-ethylmaleimide to 0.3mM final concentration followed by 1 h incubation at ambient temperature.
d) Derivatization of IgG with thiolates using site-specific functionalization of the Fc region of IgG. Site-specific introduction of thiolates into Fc region of IgG is accomplished as described in Bieniarz et al., US Patent No. 5,191,066. In that way, between 4 and 10 thiolates are introduced into Fc region of the IgG.
e) Derivatization of the Fc Thiolated IgG from part d) with maleimide derivatized PTA 24 from part a).
   The Fc thiolated IgG from part d) and maleimide derivatized PTA 24 from part a) are combined at molar ratio of 1:5 of thiolated IgG to SMTCC derivatized PTA 24. The solution is incubated in phosphate buffer pH 7.0 for 18 h at 5°C and the conjugate is purified on gel filtration column Sephadex G-25. Fractions containing proteins are pooled and concentrated using Amicon concentrator. Protein content of the final solution is established using Coomassie Dye Binding Assay, from Pierce Company.
f) Conjugation of the PTA 24 derivatized IgG from part e) to the PTA 24 derivatized calf intestinal alkaline phosphatase from part c) using double stranded 20-mer oligonucleotide composed of the complementary strands of the oligos.
   Complementary strands of oligonucleotides are hybridized and examined as described in Bioconjugate Chemistry, 4, pp. 94-102, (1993). PTA 24 derivatized IgG from part e), PTA 24 derivatized calf intestinal alkaline phosphatase and double stranded oligonucleotide 20-mer used as the conjugation template are incubated overnight at pH 7.0 for 18 h in molar ratios of 1:1:1 at ambient temperature. The conjugate is filtered through a gel filtration column G-25. The fractions are assayed for alkaline phosphatase activity using fluorogenic substrate 4-methylumbelliferyl phosphate, and for the antibody activity using the antiidiotypic IgG labelled with fluorescein.

The conjugate is also examined by gel filtration HPLC columns.

### EXAMPLE 19

### Conjugation of Intercalator Derivatized Liposome to a Double Stranded DNA

Preparation of the liposomes is carried as described in Fiechtner et al., US Patent No. 4,912,208. These liposomes display on their surface primary amines because they are prepared from diphosphatidiylethanolamine lipids. The preparation of the liposomes is carried in presence of membrane impermeable fluorescent dyes disclosed in US Patent No. 4,912,208. Thus, the molecules of the fluorescent dyes are substantially at very high, self quenching concentrations inside the liposomes and consequently they are nonfluorescent. The surface amines of the liposomes are derivatized with thiolates by essentially the same method used to introduce thiolates into alkaline phosphatase described in the EXAMPLE 18. PTA 24 and other intercalators disclosed in the preceding examples are derivatized with maleimides, utilizing SMTCC reagent, as described in EXAMPLE 18. Thiolate derivatized liposomes and maleimide derivatized PTA 24 or other intercalators are incubated together in a vessel under conditions essentially identical to the ones described in EXAMPLE 18. The fluorescent dye containing liposomes derivatized with multiple intercalators are incubated at pH ranging from 5 to 9, preferably 7.0 with the sample containing small concentrations of the double stranded DNA coated or immobilized on a solid phase. The incubation is followed by wash and addition of detergent. The concomitant lysis of the liposome membrane results in spilling of the fluorogenic dye, dequenching of the fluorescence and appearance of signal.

Alternatively, the probe multimeric single stranded DNA may be immobilized on a solid phase; the patient sample suspected to contain a complementary single stranded DNA is incubated in presence of the probe; the liposome-intercalator is brought in contact with the hybridized sample and the contents of the vessel are incubated and washed several times to remove excess of the PTA 24 derivatized liposomes. If the probe finds a complementary sequence in the patient sample, the addition of the detergent results in lysis of the liposomes and signal. If however the probe and patient sample DNA strands are not complementary, no double stranded DNA is present and substantially all the PTA 24 derivatized liposome is washed away from the solid phase, resulting in no signal.

### EXAMPLE 20

### Detection of DNA Using A Fluorescent Intercalator Conjugate Derived From Intercalator Compound 24, 85 or 90

The synthesis of intercalator maleimide functionalized intercalator can be effected by the procedure as already described in EXAMPLE 18 except that the intercalator compound

85 or 90 is used in place of PTA 24. An appropriate signal generating entity such as phycoerythrin or allophycocyanine is covalently attached to this maleimide derivatized intercalator through a thiolate on the thiolated protein prepared as described in EXAMPLE 18. After allowing time for binding of the intercalator portion of the conjugate to the immobilized ds-DNA molecule and washing away the unbound conjugate, then double stranded DNA is detected on the solid phase or other immobilized entity using the fluorescence of the phycoerythrin that is localized by the binding of the intercalator portion to the double stranded DNA molecules. The fluorescence emission is read at approximately 580 nm using methods known to those skilled in the art while exciting the fluorescent protein at a wavelength that is appropriate for efficient excitation as is determined by one skilled in the art.

### EXAMPLE 21

### Detection of DNA Using an Enzyme Intercalator Conjugate Derived From Intercalator Compound 24, 85 or 90

The synthesis of intercalator maleimide functionalized intercalator can be effected by the procedure as already described in EXAMPLE 18 except that the intercalator compound 85 or 90 is used in place of PTA 24. An appropriate signal generating entity such as alkaline phosphatase, β-galactosidase, esterase, or β-lactamase is covalently attached to this maleimide derivatized intercalator through the thiolate of the thiolated protein that is prepared as described in EXAMPLE 18. After allowing time for binding of the intercalator portion of the conjugate to the immobilized ds-DNA molecule and washing away the unbound conjugate, then double stranded DNA is detected on the solid phase or other immobilized entity such as a colloid or microparticle by fluorescence or chemiluminscence afforded by the turn-over of a non-fluorescent or non-chemiluminescent substrate of the appropriate enzyme to a fluorescent or chemiluminescent entity respectively as is determined by one skilled in the art. The fluorescence emission or chemiluminescence is then read at at the appropriate wavelengths by using standard methods of fluorescence excitation or detection of chemiluminescence respectively that are known to those skilled in the art.

### EXAMPLE 22

### Detection of DNA Using an Intercalator - Chemiluminophore Conjugate Derived from Intercalator Compound 24, 85 or 90

The synthesis of intercalator maleimide functionalized intercalator can be effected by the procedure as already described in EXAMPLE 18 except that the intercalator compound

85 or 90 is used in place of PTA 24. An appropriate activatable chemiluminescent signal generating entity such as acridinium sulfonamide is covalently attached to this intercalator by thiolate of the chemiluminescent entity that is prepared so as to be reactive towards the maleimide such as can be devised by one skilled in the art. After binding of ds-DNA with the intercalator - chemiluminophore conjugate, the excess or unbound conjugate is washed away and double stranded DNA is then detected on the solid phase or other immobilized entity by direct chemiluminscence triggered by the addition of an appropriate activation reagent such as hydrogen peroxide leading to a chemiluminescent entity and chemiluminescence is then read at at the appropriate wavelengths by using standard methods of detection of chemiluminescence such as are known to those skilled in the art.

## Claims

1. A compound having the formula a 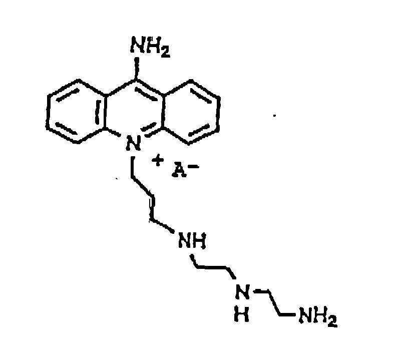 wherein A⁻ is an acceptable monovalent counter anion.

2. A compound having the formula 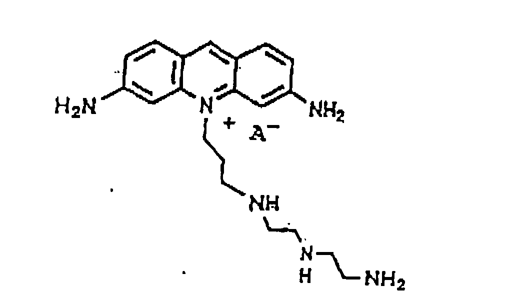 wherein A⁻ is an acceptable monovalent counter anion.

## Patentansprüche

1. Eine Verbindung, welche die folgende Formel hat 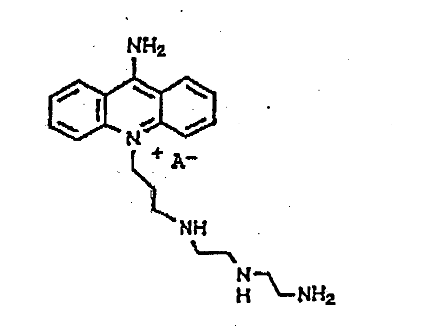 worin A⁻ ein verträgliches monovalentes Gegenanion ist.

2. Eine Verbindung, welche die folgende Formel hat 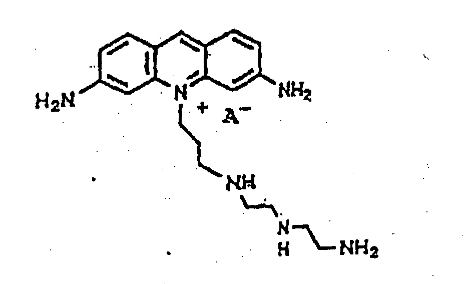 worin A⁻ ein verträgliches monovalentes Gegenanion ist.

## Revendications

1. Composé présentant la formule 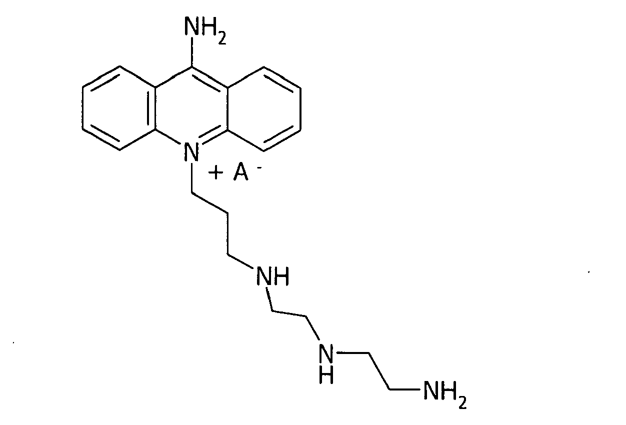 dans laquelle A⁻ est un contre-anion monovalent acceptable.

2. Composé présentant la formule 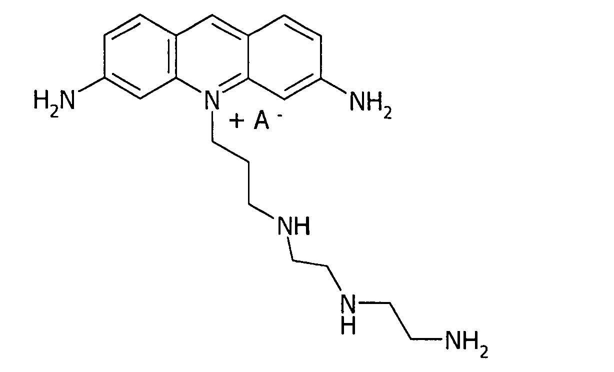 dans laquelle A⁻ est un contre-anion monovalent acceptable.
